(19) Europäisches Patentamt
European Patent Office
Office européen des brevets

(11) **EP 4 368 700 A1**

(12) **EUROPEAN PATENT APPLICATION**
published in accordance with Art. 153(4) EPC


(43) Date of publication:
**15.05.2024 Bulletin 2024/20**

(21) Application number: **22837668.7**

(22) Date of filing: **05.07.2022**

(51) International Patent Classification (IPC):
***C12N 5/071*** *(2010.01)* ***C08F 20/34*** *(2006.01)*
***C12M 1/00*** *(2006.01)*

(52) Cooperative Patent Classification (CPC):
**C08F 20/34; C12M 1/00; C12N 5/06**

(86) International application number:
**PCT/JP2022/026688**

(87) International publication number:
**WO 2023/282253 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.07.2021 JP 2021111753**



(71) Applicant: **Nissan Chemical Corporation**
**Tokyo 103-6119 (JP)**

(72) Inventors:
- **SUZUKI Kohei**
  **Funabashi-shi, Chiba 274-0052 (JP)**
- **UEDA Yuki**
  **Funabashi-shi, Chiba 274-0052 (JP)**
- **HIROI Miya**
  **Funabashi-shi, Chiba 274-0052 (JP)**
- **HIROI Yoshiomi**
  **Funabashi-shi, Chiba 274-0052 (JP)**
- **ANNO Shiho**
  **Shiraoka-shi, Saitama 349-0294 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**


(54) **CELL CULTURE BASE MATERIAL FOR SERUM-FREE MEDIUM**


(57) Objects of the present invention are to provide a base film for cell culture that enables mass production of homogeneous and high-quality cell aggregates even without the use of a biological serum, and to provide a base film-forming agent for forming the base film, and a substrate for producing cell aggregate. The present inventors provide a base film-forming agent for cell culture including a polymer, a cell adhesive substance, and a solvent, wherein the polymer contains a repeating unit derived from a monomer of formula (I) below:

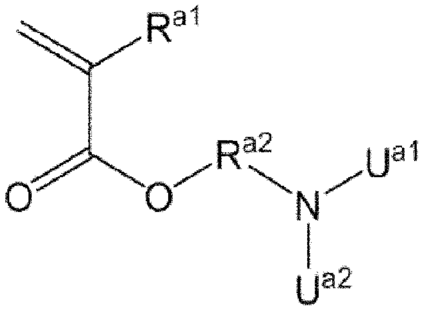

(I)

wherein $U^{a1}$, $U^{a2}$, $R^{a1}$, and $R^{a2}$ are as described in the specification and the claims.


EP 4 368 700 A1

## Description

### TECHNICAL FIELD

The present invention relates to a base film-forming agent for cell culture in a serum-free medium, a cell aggregate production substrate including the base film, and a cell aggregate production method.

### BACKGROUND ART

Various materials have been proposed as base film-forming agents for efficient cell culture.

Patent Literature 1 discloses a method for producing a polymer used as a base film for cell culture, and a cell culture vessel. Patent Literature 2 discloses a method for producing a cell structure.

The base films for cell culture in these techniques entail the use of a biological serum in order to ensure uniform adhesion of cells to the base film in the process of producing (incubating) homogeneous cell aggregates. Thus, problems are encountered, such as quality variations including variations in proliferating ability stemming from individual differences, and safety risks, for example, allergies and virus contamination by the use of serums derived from animals other than humans.

### PRIOR ART DOCUMENTS

### PATENT DOCUMENTS

Patent Document 1: WO 2020/040247
Patent Document 2: JP 2017-143755 A

### SUMMARY OF THE INVENTION

### PROBLEMS TO BE SOLVED BY THE INVENTION

Objects are to provide a base film for cell culture that enables mass production of homogeneous and high-quality cell aggregates even without the use of a biological serum, and to provide a base film-forming agent for forming it, and a substrate for producing cell aggregate.

### MEANS TO SOLVE THE PROBLEMS

The present invention includes the following.

[1] A base film-forming agent for cell culture including a polymer, a cell adhesive substance, and a solvent, wherein the polymer contains a repeating unit derived from a monomer of formula (I) below:

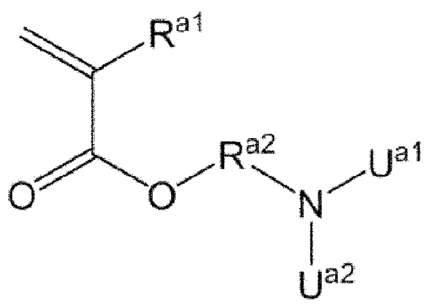

(I)

[wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group].

[2] The base film-forming agent for cell culture described in [1], wherein the polymer further contains a repeating

unit derived from a monomer of formula (II):

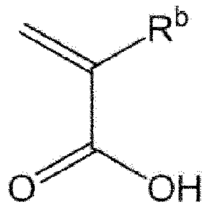

(II)

[wherein
$R^b$ denotes a hydrogen atom or a C1-C5 linear or branched alkyl group].

[3] The base film-forming agent for cell culture described in [1] or [2], wherein the weight ratio of the polymer to the cell adhesive substance is 100:0.1 to 100: 100.

[4] The base film-forming agent for cell culture described in any of [1] to [3], wherein the cell adhesive substance includes a glycoprotein.

[5] A substrate for producing cell aggregate comprising spots including a base film for cell culture formed, on a substrate having an ability to suppress adhesion of cells, by the base film-forming agent for cell culture described in any of [1] to [4].

[6] A method for producing cell aggregate including a step of forming, on a substrate having an ability to suppress adhesion of cells, a base film for cell culture including a polymer and a cell adhesive substance, wherein the polymer contains a repeating unit derived from a monomer of formula (I) below:

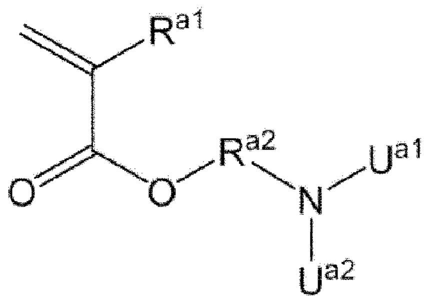

(I)

[wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group]; and a step of seeding a cell.

[7] A base film-forming agent for cell culture including a polymer, a cell adhesive substance, and a solvent, wherein the polymer contains a repeating unit of formula (Ia) below and a crosslink structure:

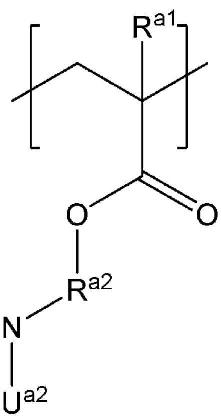

(Ia)

[wherein
$U^{a1}$ and $U^{a2}$ each independently denote a hydrogen atom or a C1-C5 linear or branched alkyl group, $R^{a1}$ denotes

a hydrogen atom or a C1-C5 linear or branched alkyl group, and $R^{a2}$ denotes a C1-C5 linear or branched alkylene group].

[8] The base film-forming agent for cell culture described in [7], wherein the crosslink structure includes a structure derived from a polyfunctional acrylate compound, a polyfunctional acrylamide compound, a polyfunctional polyester, or an isoprene compound.

[9] The base film-forming agent for cell culture described in [7], wherein the crosslink structure includes a structure of formula (IIIa), formula (IVa), and/or formula (Va) below:

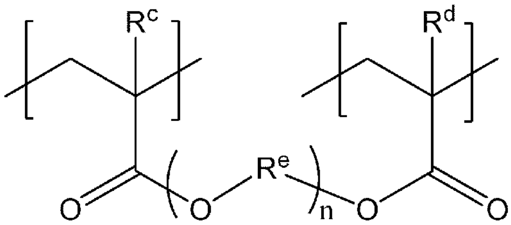

(IIIa)

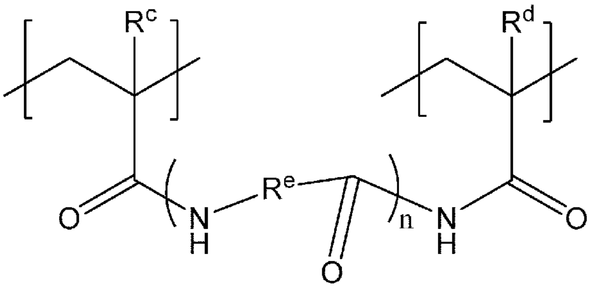

(IVa)

and/or

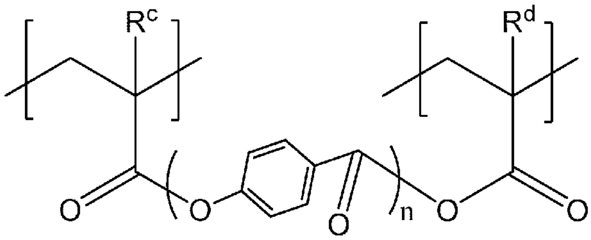

(Va)

[wherein $R^c$ and $R^d$ each independently denote a hydrogen atom or a C1-C5 linear or branched alkyl group, $R^e$ denotes a C1-C5 linear or branched alkylene group, and n denotes a number of 1 to 50].

[10] A method for producing cell aggregate including a step of forming, on a substrate having an ability to suppress adhesion of cells, a base film for cell culture including a polymer and a cell adhesive substance, wherein the polymer contains a repeating unit of formula (Ia) below and a crosslink structure:

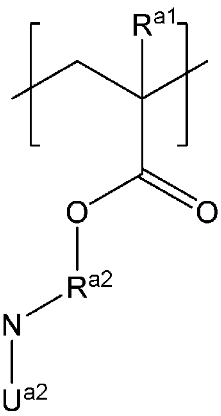

(Ia)

[wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group]; and a step of seeding a cell.

[11] A method for producing cell aggregate including a step of seeding a cell to a substrate for producing cell aggregate including a base film for cell culture including a polymer and a cell adhesive substance on a substrate having an ability to suppress adhesion of cells, wherein the polymer contains a repeating unit of formula (Ia) below and a crosslink structure:

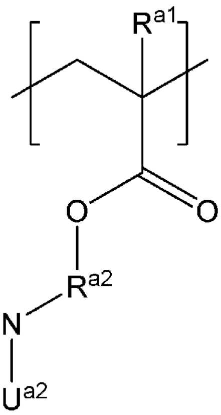

(Ia)

[wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group].

[12] A substrate for producing cell aggregate comprising spots including a base film for cell culture including a polymer and a cell adhesive substance, wherein the polymer contains a repeating unit of formula (Ia) below and a crosslink structure:

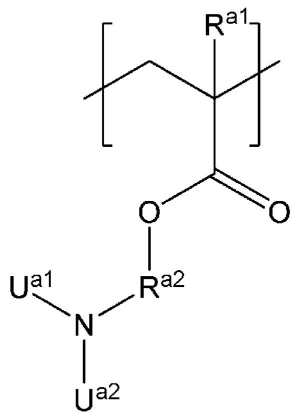

(Ia)

[wherein

$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group].

[13] A base film-forming agent for cell culture including a polymer, a cell adhesive substance, and a solvent, wherein the polymer contains a repeating unit of formula (Ia) below, a repeating unit of formula (IIa) below, and a crosslink structure:

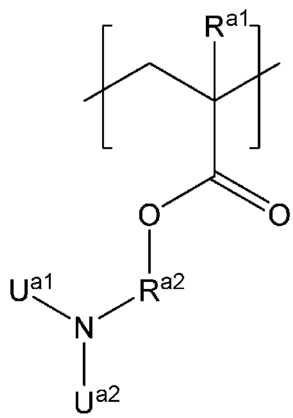

(Ia)

[wherein

$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group],

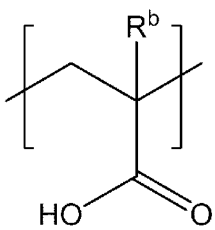

(IIa)

[wherein

$R^b$ denotes a hydrogen atom or a C1-C5 linear or branched alkyl group].

EFFECT OF THE INVENTION

A cell culture base film formed from the cell culture base film-forming agent of the present invention allows cells

to adhere uniformly to the base film under culture conditions free of animal-derived serums and thus enables production of quality cell aggregates. The use of the cell culture base film-forming agent can realize mass production of homogeneous and high-quality cell aggregates for use in the field of regenerative medicine.

BRIEF DESCRIPTION OF THE DRAWINGS

[Fig. 1] Fig. 1 is a set of stereomicroscopic images of the appearances of cell aggregate production substrates of Examples 1 to 3 and Comparative Examples 1 and 2, taken in a cell adhesion confirmation test using mouse fibroblasts in Test Example 1.

[Fig. 2] Fig. 2 is a set of stereomicroscopic images of the appearances of cell aggregate production substrates of Examples 4 and 6, taken after being left for 2 hours and for 2 days, respectively, in a cell adhesion-cell aggregate formation confirmation test using mouse fibroblasts in Test Example 2.

[Fig. 3] Fig. 3 is a set of stereomicroscopic images of the appearances of cell aggregate production substrates of Example 5 and Comparative Example 3, taken in a cell adhesion confirmation test using ADSC in Test Example 3.

[Fig. 4] Fig. 4 is a stereomicroscopic image of the appearance of a cell aggregate production substrate of Example 7, taken in a cell adhesion confirmation test using ADSC in Test Example 4.

[Fig. 5] Fig. 5 is a set of stereomicroscopic images of the appearances of cell aggregate production substrates of Examples 8 to 10, 13, 14, 17 to 19, 26, and 27, taken after being left for 2 hours and for 2 days in a cell adhesion-cell aggregate formation confirmation test using ADSC in Test Example 5.

[Fig. 6] Fig. 6 is a set of stereomicroscopic images of the appearances of cell aggregate production substrates of Examples 20 to 25 and 28 to 31, taken after being left for 2 hours and for 1 day in a cell adhesion-cell aggregate formation confirmation test using ADSC in Test Example 6.

[Fig. 7] Fig. 7 is a set of stereomicroscopic images of the appearances of cell aggregate production substrates of Examples 32 to 34, taken after being left for 6 hours and for 2 days in a cell adhesion-cell aggregate formation confirmation test using ADSC in Test Example 7.

[Fig. 8] Fig. 8 is a set of inverted microscopic images of the appearances of cell aggregate production substrates of Examples 9 and 10, taken after being left for 3 hours and for 1 day in a cell adhesion confirmation test using hiPSC in Test Example 8.

[Fig. 9] Fig. 9 is a set of inverted microscopic images of the appearances of cell aggregate production substrates of Examples 9 and 10, taken after being left for 2 days after seeding in a spheroid formation confirmation test using hiPSC in Test Example 9.

[Fig. 10] Fig. 10 is a set of inverted microscopic images of the appearances of cell aggregate production substrates of Examples 15 and 16, taken after being left for 6 hours and for 1 day after seeding in a cell adhesion confirmation test using hiPSC in Test Example 11.

[Fig. 11] Fig. 11 is a set of inverted microscopic images of the appearances of cell aggregate production substrates of Examples 15 and 16, taken after being left for 2 days after seeding in a spheroid formation confirmation test using hiPSC in Test Example 12.

[Fig. 12] Fig. 12 is a set of stereomicroscopic images of the appearance of a cell aggregate production substrate of Comparative Example 4, taken after being left for 2 hours and for 2 days in a cell adhesion-cell aggregate formation confirmation test using mouse fibroblasts in Test Example 13.

[Fig. 13] Fig. 13 is a set of stereomicroscopic images of the appearance of a cell aggregate production substrate of Comparative Example 5, taken after being left for 2 hours and for 2 days in a cell adhesion-cell aggregate formation confirmation test using mouse fibroblasts in Test Example 14.

EMBODIMENT TO CARRY OUT THE INVENTION

<Cell culture base film-forming agents>

A cell culture base film-forming agent of the present invention includes a polymer, a cell adhesive substance, and a solvent, wherein the polymer contains a repeating unit derived from a monomer of formula (I) below:

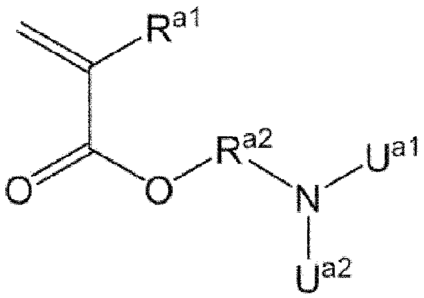

(I)

[wherein

$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group].

(Polymers)

The polymer contained in the cell culture base film-forming agent of the present application is a polymer containing a repeating unit derived from a monomer of formula (I) above.

The polymer is preferably a polymer obtained by polymerizing a cationic monomer of formula (I) with an anionic monomer of formula (II) below:

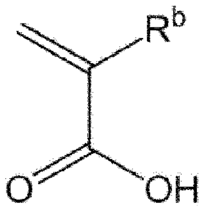

(II)

[wherein

$R^b$ denotes a hydrogen atom or a C1-C5 linear or branched alkyl group].

The polymer containing a repeating unit derived from a monomer of formula (I) may also be written as a polymer containing a repeating unit of formula (Ia) below:

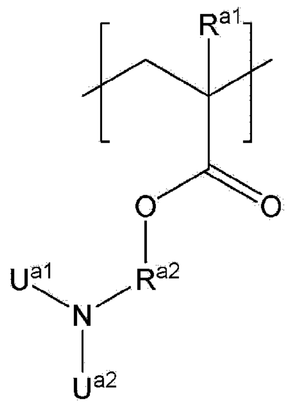

(Ia)

[wherein $U^{a1}$, $U^{a2}$, $R^{a1}$, and $R^{a2}$ are the same as defined in formula (I)]. Similarly, the polymer obtained by polymerizing a cationic monomer of formula (I) with an anionic monomer of formula (II) may also be written as a polymer containing a repeating unit of formula (Ia) above and a repeating unit of formula (IIa) below:

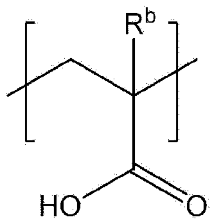

(I I a)

[wherein $R^b$ is the same as defined in formula (II)].

That is, the base film-forming agent of the present invention is a base film-forming agent that includes a polymer containing a repeating unit of formula (Ia), or a polymer containing a repeating unit of formula (Ia) and a repeating unit of formula (IIa), a cell adhesive substance, and a solvent.

In the present specification, unless otherwise defined, the "C1-C5 linear or branched alkyl group" is, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group, or a 1-ethylpropyl group.

Preferably, $R^{a1}$ and $R^b$ are each independently selected from hydrogen atom and methyl group.

Preferably, $U^{a1}$ and $U^{a2}$ are each independently selected from hydrogen atom, methyl group, ethyl group, n-propyl group, isopropyl group, and n-butyl group, are preferably each independently a methyl group or an ethyl groups, and are most preferably methyl groups.

In the present specification, unless otherwise defined, the "C1-C5 linear or branched alkylene group" may be, for example, a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, or a 1-ethyl-trimethylene group. Among these, $R^{a2}$ is preferably selected from ethylene group and propylene group.

Thus, examples of the cationic monomers of formula (I) include 2-N,N-dimethylaminoethyl methacrylate and N,N-dimethylaminomethyl methacrylate, with 2-N,N-dimethylaminoethyl methacrylate being preferable. Examples of the anionic monomers of formula (II) include acrylic acid and methacrylic acid, with methacrylic acid being preferable.

The molar ratio, units derived from the monomer of formula (I)/units derived from the monomer of formula (II), in the polymer is 100/0 to 50/50, preferably 98/2 to 50/50, more preferably 98/2 to 60/40, and particularly preferably 98/2 to 70/30.

When the molar ratio of the formula (II) is 50 or less, there is no or little decrease in cell adhesion due to the anionicity of the polymer.

(Crosslink structures)

The polymer is not particularly limited as long as it contains units derived from a monomer of formula (I) and optionally units derived from a monomer of formula (II), and may contain additional repeating units other than the units derived from the monomer(s) of formula (I)/formula (II) as long as the object of the present invention is not impaired. The polymer may contain repeating units derived from a monomer(s) of formula (I)/formula (II) in an amount of 50 mol% or more, preferably 75 mol% or more, more preferably 80 mol% or more, and still more preferably 90 mol% or more. For example, the polymer may be a polymer that includes units derived from a monomer(s) of formula (I)/formula (II), and a crosslink structure. Examples of such polymers include polymers obtained by polymerizing a monomer(s) of formula (I)/formula (II) with a monomer having two or more carbon-carbon unsaturated bonds. Specifically, the monomer having two or more carbon-carbon unsaturated bonds is a monomer having two or more carbon-carbon double bonds, with examples including polyfunctional acrylate compounds, polyfunctional acrylamide compounds, polyfunctional polyesters, and isoprene compounds.

Preferred specific examples include monomers of formulas (III) to (V) below:

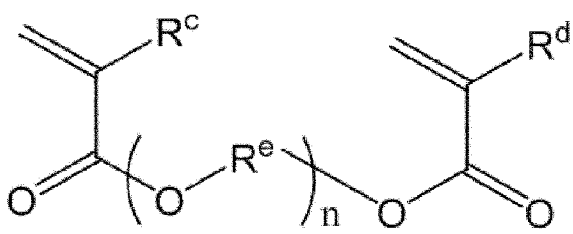

(III)

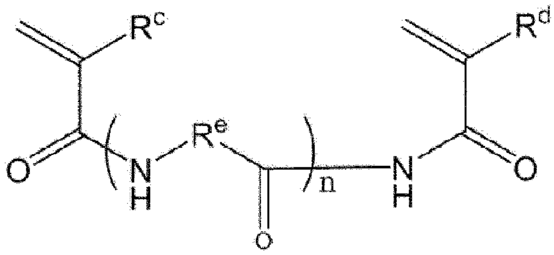

(IV)

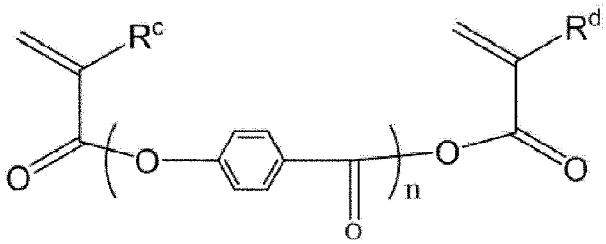

(V)

In the formulas, $R^c$ and $R^d$ each independently denote a hydrogen atom or a C1-C5 linear or branched alkyl group, $R^e$ denotes a C1-C5 linear or branched alkylene group, and n denotes a number of 1 to 50. Among these, monomers of formula (III) are preferable.

The crosslink structure may be a structure derived from a monomer having two or more carbon-carbon double bonds, and is, for example, a structure derived from a polyfunctional acrylate compound, a polyfunctional acrylamide compound, a polyfunctional polyester, or an isoprene compound, and preferably a structure derived from a monomer of any of formulas (III) to (V) above. The structure derived from a monomer of formula (III), (IV), or (V) may also be represented by formula (IIIa), (IVa), or (Va) below:

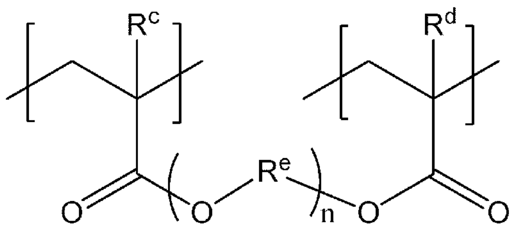

(IIIa)

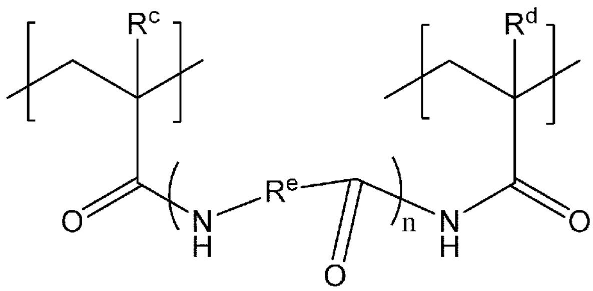

(IVa)

or

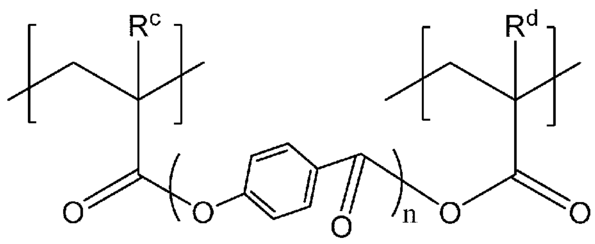

(Va)

[wherein $R^c$, $R^d$, $R^e$, and n are the same as defined in formulas (III) to (V)].

The molar ratio of the monomer represented by any of formulas (III) to (V) to the whole of the polymer is preferably 0 to 50%, and more preferably 2 to 25%.

50% or less molar ratio of any of formulas (III) to (V) eliminates or reduces the occurrence of gelation of solids during the production due to the increase in molecular weight caused by excessive crosslinking, thereby facilitating the production.

Preferably, $R^c$ and $R^d$ are each independently selected from hydrogen atom and methyl group.

$R^e$ is preferably selected from methylene group, ethylene group, and propylene group, and is most preferably an ethylene group.

The letter n is a number of 1 to 50. The letter n is preferably a number of 1 to 30. The letter n is preferably a number of 1 to 10.

The difference is 0 to 10 mol% between the value of mol% of the monomer represented by formula (II) relative to the whole of the polymer and the value of mol% of the monomer represented by formula (II) relative to the total amount of the monomers charged in the preparation process. The polymer of the present application produced by the method described later has a small difference between the ratio of the monomer charged and the value measured with respect to the polymer produced, and the difference is 0 to 10 mol%, and more preferably 0 to 8 mol%.

The number average molecular weight (Mn) of the polymer is 20,000 to 1,000,000, and more preferably 50,000 to 800,000.

The ratio (Mw/Mn) of the weight average molecular weight (Mw) to the number average molecular weight (Mn) of the polymer is 1.01 to 10.00, preferably 1.2 to 8.0, preferably 1.4 to 6.0, preferably 1.5 to 5.0, and preferably 1.6 to 4.5.

For example, the number average molecular weight (Mn) and the number average molecular weight (Mn) may be determined by gel filtration chromatography described in Examples.

The polymer of the present application used as a cell culture base film allows cells to adhere thereto and detach therefrom later to form cell aggregates. The term cell aggregates indicates structures formed as a result of aggregation of cells, and the shape is not limited and may be, for example, spherical or ring. The number of types of the cells constituting the cell aggregates is not limited, and the cell aggregates may be composed of a plurality of types of cells. Examples of the cell aggregates include various structures, such as spheroids, organ primordia, and organoids. Compared to cell aggregates prepared by non-adhesive culture on conventional low-cell adhesion plates, advantages are obtained in, for example, control of the size of cell aggregates by defining the adhesion area (cell aggregates can be produced with a desired size).

The entire contents disclosed in WO 2020/040247 and Japanese Patent Application No. 2020-028120 are incorporated herein by reference.

(Cell adhesive substances)

The cell culture base film-forming agent of the present invention includes a cell adhesive substance. The incorporation of a cell adhesive substance makes it possible to promote the adhesion, the stretching, the proliferation, and the differentiation of cells.

The cell adhesive substance that is used may be a known substance, for example, a biological substance, such as an extracellular matrix (ECM) protein, a glycoprotein, or a peptide, or a synthetic compound (low-molecular weight or high-molecular weight). A compound that is not a biological substance, for example, a synthetic compound (low-molecular weight or high-molecular weight) is preferable. The term low-molecular weight means that, for example, the weight average molecular weight of the compound is 2,000 or less. The term high-molecular weight means that, for example, the weight average molecular weight is 2,000 or more, and the upper limit is, for example, 1,000,000.

Examples of the extracellular matrix (ECM) proteins include collagen (for example, type I collagen (product numbers C9791, C7661, C1809, C2249, and C2124), type II collagen (product number C9301), and type IV collagen (product numbers C0543 and C5533) from Merck), elastin (for example, Merck product numbers: E1625 and E6527), fibronectin (for example, Merck product numbers F1141, F0635, F2518, F0895, F4759, and F2006), laminin (for example, Merck product numbers: L6724, L2020, and L4544), laminin fragments (for example, 892011 from Matrixome), and

vitronectin (for example, VTN-N (Gibco), Vitronectin, Human, Recombinant, Animal Free (PeproTech), and Merck product numbers: V0132, V9881, V8379, 08-126, and SRP3186).

The cell adhesive substance is preferably a glycoprotein. Specifically, the cell adhesive substance is preferably selected from vitronectin, integrin, cadherin, fibronectin, laminin, tenascin, osteopontin, and bone sialoprotein. The cell adhesive substance is preferably a protein that has an RGD sequence as an amino acid sequence.

Examples of the peptides include ECM peptide (MAPTrix (registered trademark) from Kollodis Bio Sciences) and RGD peptide (180-01531 from FUJIFILM Wako Pure Chemical Corporation).

Examples of the synthetic compounds (high-molecular weight) include polylysine (for example, Merck products: P4707, P4832, P7280, P9155, P6407, P6282, P7405, and P5899) and polyornithine (for example, Merck product number P4975). Examples of the synthetic compounds (low-molecular weight) include adhesamine (for example, AD-00000-0201 from NAGASE & CO., LTD.) and synthetic cyclic RGD peptide (for example, LS-3920.0010 from IRIS BIOTECH).

The ratio (by mass) of the polymer to the cell adhesive substance in the cell culture base film-forming agent of the present invention is not limited as long as the base-forming agent that is formed is capable of cell culture, but is preferably 100:0.1 to 100:100. When the ratio of the cell adhesive substance is 0.1 or more, sufficient cell adhesion is exhibited. When the ratio of the cell adhesive substance is 100 or less, cells that have adhered are allowed to aggregate (to form cell aggregates) easily.

The cell culture base film-forming agent of the present invention includes a solvent. The solvent is not limited as long as being capable of dissolving the polymer. A water-containing solution, including water, is preferable.

Examples of the water-containing solutions include water, salt-containing aqueous solutions, such as saline and phosphate-buffered solutions, and mixed solvents combining water or a salt-containing aqueous solution with an alcohol. Examples of the alcohols include C2-C6 alcohols, such as ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (= neopentyl alcohol), 2-methyl-1-propanol, 2-methyl-1-butanol, 2-methyl-2-butanol (= t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol, and cyclohexanol. The mixed solvent may contain a single or a combination of these alcohols.

For example, the content of water in the water-containing solution is 50 mass% to 100 mass%, 80 mass% to 100 mass%, or 90 mass% to 100 mass%.

In addition to the polymer, the cell adhesive substance, and the solvent, the base film-forming agent may further include additional substances as required while ensuring that the performance of a base film that is obtained will not be impaired. Examples of the additional substances include pH adjusters, crosslinking agents, preservatives, surfactants, primers to enhance the adhesion to vessels or substrates, antifungal agents, and sugars.

(Cells)

A cell in the present invention is the most basic unit constituting an animal or a plant, and has a cytoplasm and various kinds of organelles as its elements inside the cell membrane. Here, the nucleus containing DNA may or may not be contained inside the cell. Examples of animal-derived cells in the present invention include germ cells, such as sperm and ovum, somatic cells constituting the living body, stem cells (such as pluripotent stem cells), progenitor cells, cancer cells separated from the living body, cells that have been separated from the living body and have acquired an immortalization potential and are stably maintained in vitro (cell lines), cells that have been separated from the living body and have undergone artificial genetic modification, and cells that have been separated from the living body and have undergone artificial nucleus exchange. Examples of the somatic cells constituting the living body include, but are not limited to, fibroblasts, bone marrow cells, B lymphocytes, T lymphocytes, neutrophils, red blood cells, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells and skeletal muscle cells), pancreatic beta cells, melanocytes, hematopoietic progenitor cells (for example, CD34-positive cells derived from umbilical cord blood), and mononuclear cells. Examples of the somatic cells include cells collected from any tissues, such as skin, kidney, spleen, adrenal gland, liver, lung, ovary, pancreas, uterus, stomach, colon, small intestine, large intestine, bladder, prostate, testis, thoracic gland, muscle, connective tissue, bone, cartilage, blood vessel tissue, blood (including umbilical cord blood), bone marrow, heart, myocardium, eye, brain, and nerve tissue. Examples of the somatic cells further include cells induced to differentiate from stem cells or progenitor cells.

Stem cells are cells that have an ability to replicate themselves and an ability to differentiate into various types of cells. Examples thereof include, but are not limited to, embryonic stem cells (ES cells), embryonic tumor cells, embryonic germline stem cells, induced pluripotent stem cells (iPS cells), neural stem cells, hematopoietic stem cells, mesenchymal

stem cells, liver stem cells, pancreatic stem cells, muscle stem cells, germline stem cells, intestinal stem cells, cancer stem cells, and hair follicle stem cells. Examples of the pluripotent stem cells include ES cells, embryonic germline stem cells, and iPS cells belonging to the above stem cells. The progenitor cells are cells in the course of differentiation from the stem cells into specific somatic cells or germ cells. The cancer cells are cells that are derived from somatic cells and have acquired an infinite proliferative capacity. The cell lines are cells that have acquired an infinite proliferative capacity by artificial manipulation in vitro. Among these, fibroblasts and stem cells are preferable. Among the stem cells, pluripotent stem cells are more preferable.

<Substrate for producing cell aggregate>

A substrate for producing cell aggregate of the present invention includes a spot including a base film for cell culture formed, on a substrate having an ability to suppress adhesion of cells, by the base film-forming agent for cell culture.

The substrate for producing cell aggregate of the present invention is produced using a substrate having an ability to suppress adhesion of cells. The substrate may be subjected to the treatment to suppress adhesion of cells before the formation of the spot (the base film). For example, the substrate having an ability to suppress adhesion of cells may be a commercially available cell culture dish with low-cell adhesion treatment, or a commercially available culture vessel having an ability to suppress adhesion of cells. For example, the cell culture vessel described in Japanese Patent Application Kokai Publication No. 2008-61609 may be used. However, the substrate is not limited thereto. For example, the substrate may be one that is produced through a step in which a known composition for forming a coating film having an ability to suppress adhesion of cells is applied. For example, the composition for forming a coating film used here may be the composition for forming a coating film described in WO 2014/196650. Preferably, the composition for forming a coating film includes a solvent and a copolymer (P) that contains a repeating unit including an organic group of formula (a) below and a repeating unit including an organic group of formula (b) below:

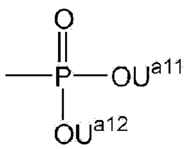

(a)

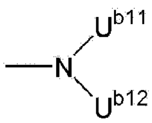

or

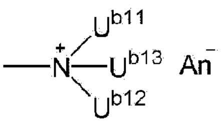

(b)

[wherein

$U^{a11}$, $U^{a12}$, $U^{b11}$, $U^{b12}$, and $U^{b13}$ each independently denote a hydrogen atom or a C1-C5 linear or branched alkyl group, and $An^-$ denotes an anion selected from the group consisting of halide ion, inorganic acid ion, hydroxide ion, and isothiocyanate ion], and the composition for forming a coating film is applied to the surface of a vessel or a substrate and is dried. The coating film is formed on at least part of the surface of the substrate, and is preferably formed over the entire side on which cell aggregates will be produced (that is, the side on which a spot of the present application will be formed) or over the entire surface of the substrate.

The entire contents disclosed in WO 2014/196650 and WO 2016/093293 are incorporated herein by reference.

For example, the term "having an ability to suppress adhesion of cells" means that the relative absorbance (WST O.D. 450 nm) (%) compared to the absorbance in the absence of the coating film or without the low-cell adhesion treatment ((absorbance of Example (WST O.D. 450 nm))/(absorbance of Comparative Example (WST O.D. 450 nm))) as measured with a fluorescence microscope in accordance with the method described in Examples of WO 2016/093293 is 50% or less, preferably 30% or less, and more preferably 20% or less.

As the copolymer for forming a coating film having an ability to suppress adhesion of cells, it may further be used include one obtained by copolymerizing an ethylenically unsaturated monomer, or a polysaccharide or a derivative thereof. For example, the ethylenically unsaturated monomer may be one, or two or more ethylenically unsaturated monomers selected from the group consisting of (meth)acrylic acid and esters thereof; vinyl acetate; vinylpyrrolidone; ethylene; vinyl alcohol; and hydrophilic functional derivatives thereof. Examples of the polysaccharides and the derivatives thereof include cellulose polymers, such as hydroxyalkylcelluloses (for example, hydroxyethylcellulose and hydroxypropylcellulose), starches, dextrans, and curdlans.

The hydrophilic functional derivatives are ethylenically unsaturated monomers having a hydrophilic functional group or structure. Examples of the hydrophilic functional groups or structures include betaine structures; amide structures; alkylene glycol residues; amino groups; and sulfinyl groups.

The betaine structures are monovalent or divalent groups derived from compounds having an amphoteric center of a quaternary ammonium-type cation structure and an acidic anion structure, with examples including phosphorylcholine group:

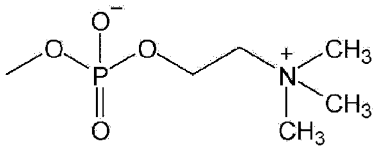

Examples of the ethylenically unsaturated monomers having such a structure include 2-methacryloyloxyethylphosphorylcholine (MPC).

The amide structures are groups of the formula below:

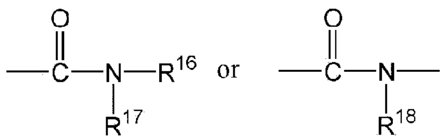

[wherein $R^{16}$, $R^{17}$, and $R^{18}$ each independently denote a hydrogen atom or an organic group (such as, for example, a methyl group, a hydroxymethyl group, or a hydroxyethyl group)]. Examples of the ethylenically unsaturated monomers having such a structure include (meth)acrylamide, N-(hydroxymethyl)(meth)acrylamide, and N-isopropyl(meth)acrylamide. Furthermore, monomers or polymers having such a structure are disclosed in, for example, Japanese Patent Application Kokai Publication No. 2010-169604.

The alkylene glycol residues are alkyleneoxy groups (-Alk-O-) that remain after a hydroxyl group(s) at one end or both ends of an alkylene glycol (HO-Alk-OH where Alk is a C1-C10 alkylene group) undergo(es) condensation reaction with other compound, and include poly(alkyleneoxy) groups in which alkyleneoxy units are repeated. Examples of the ethylenically unsaturated monomers having such a structure include 2-hydroxyethyl (meth)acrylate and methoxypolyethylene glycol (meth)acrylate. Furthermore, monomers or polymers having such a structure are disclosed in, for example, Japanese Patent Application Kokai Publication No. 2008-533489.

The amino group is a group of the formula: $-NH_2$, $-NHR^{19}$, or $-NR^{20}R^{21}$ [where $R^{19}$, $R^{20}$, and $R^{21}$ each independently denote an organic group (such as, for example, a C1-C5 linear or branched alkyl group)]. The amino groups in the present invention include quaternized or chlorinated amino groups. Examples of the ethylenically unsaturated monomers having such a structure include dimethylaminoethyl (meth)acrylate, 2-(t-butylamino)ethyl (meth)acrylate, and methacryloylcholine chloride.

The sulfinyl group is a group of the formula below:

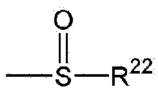

[wherein $R^{22}$ is an organic group (such as, for example, a C1-C10 organic group, preferably a C1-C10 alkyl group having one or more hydroxyl groups)]. Examples of the polymers having such a structure include copolymers disclosed in, for example, Japanese Patent Application Kokai Publication No. 2014-48278.

The coating film having an ability to suppress adhesion of cells may include a water-insoluble copolymer that is hardly dissolved in a phosphate-buffered saline.

In the present specification, the term "water-soluble" means that 1.0 g or more can be dissolved in 100 g of water at 25°C. The term "water-insoluble" means that the solubility does not correspond to "water-soluble", that is, the solubility in 100 g of water at 25°C is less than 1.0 g. Thus, the term "water-insoluble copolymer" means a copolymer having a solubility of less than 1.0 g in 100 g of water at 25°C, in particular, a copolymer having a solubility of less than 1.0 g in 100 g of a phosphate-buffered saline at 25°C.

Examples of the water-insoluble copolymers include a copolymer containing a repeating unis (A) of formula (A) below and a repeating unit (B) of formula (B) below.

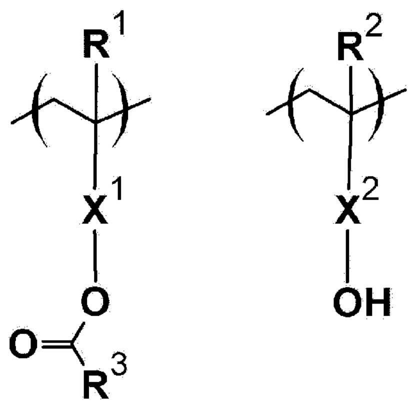

(A) (B)

In the formulas, $R^1$ to $R^3$ each independently denote a hydrogen atom or a C1-C5 linear or branched alkyl group, and $X^1$ and $X^2$ each independently denote a single bond, an ester bond, an ether bond, an amide bond, or a C1-C5 linear or branched alkylene group optionally interrupted by an oxygen atom.
The water-insoluble copolymer may contain two or more kinds of repeating units (A) and may contain two or more kinds of repeating units (B), but preferably contains a single kind of repeating units (A) and a single kind of repeating units (B).
In the water-insoluble copolymer, it is preferable that $R^1$ to $R^3$ each independently denote a hydrogen atom, a methyl group, or an ethyl group.
In the present specification, unless otherwise defined, the "ester bond" means - C(=O)-O- or -O-C(=O)-, the "ether bond" means -O-, and the "amide bond" means - NHC(=O)- or -C(=O)NH-.
In the present specification, unless otherwise defined, the term "C1-C5 linear or branched alkylene group optionally interrupted by an oxygen atom" means a C1-C5 linear or branched alkylene group, or a C1-C5 linear or branched alkylene group in which one, or two or more pairs of carbon atoms are bonded via an ether bond.
In the water-insoluble copolymer, it is preferable that $X^1$ and $X^2$ each independently denote a methylene group, an ethylene group, or a propylene group.
In the water-insoluble copolymer, it is preferable that $R^1$ and $R^2$ be hydrogen atoms, $R^3$ be a methyl group, and $X^1$ and $X^2$ be single bonds.
The molar ratio (A:B) of the repeating units (A) to the repeating units (B) in the water-insoluble copolymer is 89:11 to 50:50.
When the total number of moles of the repeating units (A) and the repeating units (B) in the water-insoluble copolymer is taken as 100, the molar ratio (A:B) of the repeating units (A) to the repeating units (B) can be written as (100 - m):m. In this case, the range of m is 11 to 50. The lower limit of m may be 12, 13, 14, 15, 16, 17, 18, 19, 20, 21, 22, 23, 24, 25, 26, 27, 28, 29, or 30. The upper limit of m may be 49, 48, 47, 46, 45, 44, 43, 42, 41, 40, 38, 37, 36, or 35. The range of m is, for example, 12 to 49, 12 to 48, 15 to 48, 20 to 49, 20 to 45, 22 to 49, or 22 to 45.
The mol% of the total of the repeating units (A) and the repeating units (B) in all the repeating units in the water-insoluble copolymer is not particularly limited but is preferably 90 mol% or more, more preferably 95 mol% or more, still more preferably 99.5 mol% or more, and particularly preferably 100%.
By controlling the molar ratio of the repeating units (A) and the repeating units (B) in the water-insoluble copolymer to the specific range, a coating film hardly soluble in a phosphate-buffered saline can be obtained without crosslinking of the copolymer. Thus, the water-insoluble copolymer requires no photosensitive groups for crosslinking of the copolymer. That is, the water-insoluble copolymer is preferably free of photosensitive groups. Examples of the photosensitive groups include azide groups. Because the water-insoluble copolymer requires no photosensitive groups for crosslinking of the copolymer, a coating film can be formed without the need of photoirradiation for crosslinking of the copolymer. Thus, the use of the water-insoluble copolymer can simplify the process for forming a coating film having an ability to suppress adhesion of cells.
The viscosity average polymerization degree (hereinafter, sometimes written simply as the "polymerization degree") of the water-insoluble copolymer is not particularly limited but is preferably 200 to 3,000, more preferably 200 to 2,500, and particularly preferably 200 to 2,000 in order to obtain an ability to suppress adhesion of cells favorably.
The viscosity average polymerization degree is measured with respect to the water-insoluble copolymer completely saponified.
The "viscosity average polymerization degree" of polyvinyl alcohol obtained by complete saponification is a

value calculated from the equation below using the intrinsic viscosity [η] (g/dL) measured at 30°C with an Ostwald viscometer using ion-exchanged water as a solvent.

[Math. 1]

$$\log(P) = 1.613 \times \log([\eta] \times 10^4 / 8.29)$$

Here, P indicates the viscosity average polymerization degree.

The viscosity average polymerization degree may be determined in accordance with JIS K6726.

The water-insoluble copolymer may be produced by any method without limitation. In an exemplary method, a compound of formula (C) below is polymerized to produce a homopolymer, and the homopolymer obtained is partially hydrolyzed by known saponification reaction to give a copolymer.

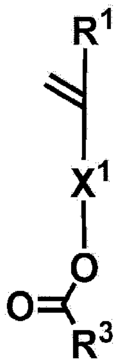

(C)

In the formula, $R^1$, $R^3$, and $X^1$ are the same as defined hereinabove.

Alternatively, for example, the water-insoluble copolymer may be produced by a method in which a compound of formula (C) below and a compound of formula (D) below are copolymerized to give a copolymer.

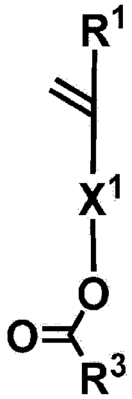

(C)

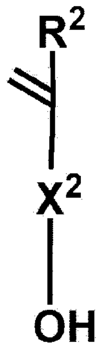

(D)

In the formulas, $R^1$ to $R^3$, $X^1$, and $X^2$ are the same as defined hereinabove.

The water-insoluble copolymer may be a random copolymer or a block copolymer.

The water-insoluble copolymer that is used may be a commercially available product. Specific examples of the commercially available copolymers include polyvinyl acetate (product name: JMR-150L (registered trademark) manufactured by JAPAN VAM & POVAL CO., LTD.).

In the composition for the coating film that is used in the production of the substrate having an ability to suppress adhesion of cells, according to the present invention, the content of the copolymer in the film-forming components is not particularly limited but is preferably 80 mass% or more, more preferably 90 mass% or more, and particularly preferably 95 mass% or more. The film-forming components are components constituting the composition except the solvent component.

In the composition for the coating film that is used in the production of the substrate having an ability to suppress

adhesion of cells, according to the present invention, the content of the copolymer is not particularly limited but, from the point of view of facilitating the formation of a coating film with a desired thickness, is preferably 0.1 to 10 mass%, more preferably 0.3 to 8 mass%, and particularly preferably 0.5 to 5 mass%. The content of the copolymer in the coating film-forming composition may be 0.02 to 2 mass% or may be 0.05 to 1 mass%.

(Spots)

The cell aggregate production substrate of the present invention includes a spot, preferably a plurality of spots (a base film). The ratio of the total area of the spots, the diameter of each spot, and the center-to-center interval between the spots may be selected appropriately from predetermined ranges in accordance with factors, such as the types of the cells and the substrate that are used, and the desired size of cell aggregates. The ratio of the total area of the spots relative to the surface area of the substrate is preferably 30% or more, 40% or more, or 50% or more, and is preferably 99% or less. The diameter of each spot is 50 to 5000 μm, and preferably 100 to 3000 μm. The center-to-center interval between the spots is 100 to 6000 μm, 150 to 4000 μm, and preferably 150 to 300 μm.

In the present invention, independent cell-adhesive micro-sized regions (spots) are arranged with a high density, preferably regularly, on the substrate having an ability to suppress adhesion of cells. In this manner, a plurality of spheroids having a uniform size can be formed on the single substrate (vessel) at one time.

The spots may be formed by applying the base film-forming agent. For example, the base film-forming agent may be applied by an inkjet method, a screen printing method, a slit coating method, or a roll-to-roll method. Preferably, a printing technique, such as inkjet printer or screen printing, is used.

The application may be performed by other methods. For example, a method may be adopted in which a substrate optionally after protection of regions other than the spot formation scheduled regions is immersed in the base film-forming agent, or the base film-forming agent is added to a substrate (a vessel) optionally after protection of regions other than the spot formation scheduled regions and is allowed to stand for a predetermined time. In an embodiment in which the substrate is a cell culture vessel, the application is performed by a method in which the base film-forming agent is added to the vessel optionally after protection of regions other than the spot formation scheduled regions and is allowed to stand for a predetermined time. For example, the addition may be performed in such a manner that the base film-forming agent in an amount 0.5 to 1 time the total volume of the vessel is added using a syringe or the like. The agent is allowed to stand for a time and at a temperature that are selected appropriately in accordance with the material of the vessel or the substrate and the type of the cell culture base film-forming agent. For example, the agent is allowed to stand at 10 to 80°C for 1 minute to 24 hours, preferably 5 minutes to 3 hours. A cell aggregate production substrate can be thus produced.

The substrate obtained by the above-described method may be used as a cell aggregate production substrate directly without a step of drying the spots on the surface of the substrate or after the spots are washed with water or the medium of a sample that will be subjected to cell culture (such as, for example, water, buffer solution, or culture medium).

Specifically, the substrate may be used as a cell aggregate production substrate directly without a drying step or after the spots are washed with water or the medium of a sample that will be subjected to cell culture (such as, for example, water, buffer solution, or culture medium, particularly preferably culture medium (for example, DMEM medium (Dulbecco's modified eagle medium))), within 48 hours, preferably within 24 hours, more preferably within 12 hours, still more preferably within 6 hours, further preferably within 3 hours, and still further preferably within 1 hour after the formation of spots on the surface of the substrate.

The cell aggregate production substrate may be subjected to a drying step. The drying step is performed in the air or under vacuum, preferably at a temperature in the range of -200°C to 200°C. The drying step removes the solvent in the base film-forming agent, and the base film-forming agent is completely fixed to the substrate.

For example, the spots may be formed by drying at room temperature (10°C to 35°C, preferably 20°C to 30°C, for example, 25°C). In order to accelerate the spot formation, for example, the drying may be performed at 40°C to 80°C. Drying at a temperature below -200°C entails the use of a less common refrigerant and is thus not versatile, and is also inefficient because the drying requires a long time due to solvent sublimation. If the drying temperature is above 200°C, the polymer is thermally decomposed. The drying temperature is more preferably 10°C to 180°C, and the drying temperature is more preferably 20°C to 150°C. The cell aggregate production substrate of the present application is produced through the simple steps described above.

In order to remove impurities, free polymer, and the like that remain in the spots (the base film), a step may be performed in which the substrate is washed with at least one solvent selected from water and aqueous electrolyte solutions. For example, the washing is desirably washing with running water or ultrasonic washing. The water or the aqueous electrolyte solution may be heated in the range of, for example, 40°C to 95°C. The aqueous electrolyte solution is preferably PBS, saline (containing only sodium chloride), Dulbecco's phosphate-buffered saline, Tris-buffered saline, HEPES-buffered saline, or Veronal-buffered saline, and is particularly preferably PBS. After the agent is fixed, the coating film is not dissolved and remains firmly fixed to the substrate even when washed with, for example, water, PBS, or alcohol.

The film thickness of the spots (the base film) of the present application is such that the maximum film thickness and the minimum film thickness are in the range of 1 to 1000 nm, preferably in the range of 5 to 500 nm.

(Substrates)

The cell aggregate production substrate of the present invention may be produced by applying the base film-forming agent to the surface of the substrate and drying the coating. Here, the term "surface" indicates a face that will be brought into contact with the content, such as cells or a cell culture medium.

The shape of the substrate surface may be flat or irregular, and is preferably flat.

Examples of the materials of the substrates include glass, metals, metal-containing compounds or metalloid-containing compounds, activated carbon, and resins. Examples of the metals include typical metals: (aluminum group elements: Al, Ga, In; iron group elements: Fe, Co, Ni; chromium group elements: Cr, Mo, W, U; manganese group elements: Mn, Re; and noble metals: Cu, Ag, Au. Examples of the metal-containing compounds and the metalloid-containing compounds include ceramics that are based on metal oxides and are sintered bodies baked by heat treatment at a high temperature; semiconductors, such as silicon; inorganic solid materials, such as shaped products of inorganic compounds, for example, metal oxides or metalloid oxides (such as silicon oxide and alumina), metal carbides or metalloid carbides, metal nitrides or metalloid nitrides (such as silicon nitride), and metal borides or metalloid borides; aluminum, nickel-titanium, and stainless steel (such as SUS304, SUS316, and SUS316L).

The resins may be natural resins or derivatives thereof, or synthetic resins. Preferred examples of the natural resins and the derivatives thereof include cellulose, cellulose triacetate (CTA), nitrocellulose (NC), and dextran sulfate cellulose. Preferred examples of the synthetic resins include polyacrylonitrile (PAN), polyimide (PI), polyester-based polymer alloys (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyesters, polypropylene (PP), polyvinylidene fluoride (PVDF), polyether sulfone (PES), polycarbonate (PC), cycloolefin polymers (COP), polyvinyl chloride (PVC), polytetrafluoroethylene (PTFE), ultrahigh molecular weight polyethylene (UHPE), polydimethylsiloxane (PDMS), acrylonitrile-butadiene-styrene resin (ABS), and Teflon (registered trademark).

In the production of the cell aggregate production substrate of the present invention, even a material that is low in heat resistance, for example, a resin, may be used because the base film formation does not require a high-temperature treatment.

The substrate may be composed of a single material or a combination of two or more kinds of materials. In order to ensure that, for example, the cell aggregate production substrate of the present application is applicable to mass production of cell culture aggregates, the substrate may be a flexible substrate that can be wound up (rolled) similarly to a belt conveyor. Examples of the materials of such rollable substrates include synthetic resins and natural polymers.

The substrate of the present application may be a substrate used as a so-called cell culture vessel. Examples thereof include dishes or Schale dishes, such as Petri dishes commonly used for cell culture, tissue culture dishes, and multi-dishes; flasks, such as cell culture flasks, spinner flasks, and multi-layered flasks; bags, such as plastic bags, Teflon (registered trademark) bags, and culture bags; plates, such as microplates, microwell plates, multiplates, and multiwell plates; chamber slides; tubes; trays; and bottles, such as roller bottles.

<Method for producing cell aggregate>

A method for producing cell aggregate of the present invention includes a step of forming, on a substrate having an ability to suppress adhesion of cells, a base film for cell culture including a polymer and a cell adhesive substance, wherein the polymer contains a repeating unit derived from a monomer of formula (I) below:

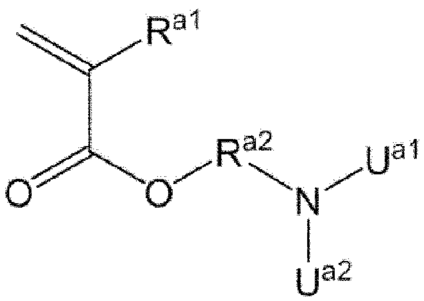

(I)

[wherein

$U^{a1}$ and $U^{a2}$ each independently denote a hydrogen atom or a C1-C5 linear or branched alkyl group, $R^{a1}$ denotes a

hydrogen atom or a C1-C5 linear or branched alkyl group, and $R^{a2}$ denotes a C1-C5 linear or branched alkylene group]; and a step of seeding a cell and culturing the cell. The cell seeding and culturing step is not particularly limited and may be performed in a known appropriate manner depending on the type of the cells. Details of the substrate having an ability to suppress adhesion of cells, the step of forming (the method for producing) the base film, and the cells are as described hereinabove.

The cell aggregate production method of the present invention is advantageous in that the cell seeding and culturing step can be performed in the presence or absence of a biological serum, and, in particular, high-quality cell aggregates can be produced even when the culture medium has a low concentration (for example, less than 5 mass%, in particular, less than 3 mass%) of a biological serum or contains no biological serums.

EXAMPLES

Hereinbelow, the present invention will be described in greater detail based on Examples and Comparative Examples. However, the present invention is not limited to Examples described below.

<Method for measuring weight average molecular weight>

The weight average molecular weights described in Synthesis Examples below are results obtained by gel filtration chromatography (hereinafter, abbreviated as GFC).

(Measurement conditions)

- Device: HLC-8320GPC (manufactured by TOSOH CORPORATION)
- GFC columns: TSKgel G6000 + 3000 PWXL-CP
- Flow rate: 1.0 mL/min
- Eluent: Salt-containing water/organic mixed solvent
- Column temperature: 40°C
- Detector: RI
- Injection concentration: 0.05 mass% polymer solid content
- Injection volume: 100 μL
- Calibration curve: Cubic approximation curve
- Standard samples: Polyethylene oxide (manufactured by Agilent) × 10 types

<Synthesis Example 1>

24.00 g of 2-(dimethylamino)ethyl methacrylate (manufactured by Tokyo Chemical Industry Co., Ltd.), 1.46 g of methacrylic acid (manufactured by Tokyo Chemical Industry Co., Ltd.), 5.09 g of ethylene glycol dimethacrylate (manufactured by Tokyo Chemical Industry Co., Ltd.), 0.31 g of dimethyl 1,1'-azobis(1-cyclohexanecarboxylate) (VE-073 manufactured by FUJIFILM Wako Pure Chemical Corporation), and 111.09 g of 2-propanol were mixed together. The mixture was dropped to 166.62 g of 2-propanol at a reflux temperature to perform dropwise polymerization. A polymer was thus synthesized. The reaction product was reprecipitated with poor solvent hexane, and the precipitate was recovered by filtration and was dried under reduced pressure.

The weight average molecular weight of this polymer according to GFC was 228,000 (hereinafter, written as "Synthesis Example Polymer 1").

<Synthesis Example 2>

8.00 g of 2-(dimethylamino)ethyl methacrylate (manufactured by Tokyo Chemical Industry Co., Ltd.), 1.88 g of methacrylic acid (manufactured by Tokyo Chemical Industry Co., Ltd.), 1.98 g of ethylene glycol dimethacrylate (manufactured by Tokyo Chemical Industry Co., Ltd.), 0.12 g of dimethyl 1,1'-azobis(1-cyclohexanecarboxylate) (VE-073 manufactured by FUJIFILM Wako Pure Chemical Corporation), and 43.10 g of 2-propanol were mixed together. The mixture was dropped to 64.65 g of 2-propanol at a reflux temperature to perform dropwise polymerization. A polymer was thus synthesized. The reaction product was reprecipitated with poor solvent hexane, and the precipitate was recovered by filtration and was dried under reduced pressure.

The weight average molecular weight of this polymer according to GFC was 438,000 (hereinafter, written as "Synthesis Example Polymer 2").

<Preparation Example 1>

Polyvinyl acetate (JMR-150L (registered trademark) manufactured by JAPAN VAM & POVAL CO., LTD. (polymerization degree: 1480, saponification degree: 22.7%)) was dissolved into ethanol/1-methoxy-2-propanol (7/3 by mass) so that the concentration thereof would be 10 mg/g. A composition for forming a coating film 1 having an ability to suppress adhesion of cells was thus prepared. The composition obtained was transparent and uniform.

<Preparation Example 2>

Polyvinyl acetate (JMR-150L (registered trademark) manufactured by JAPAN VAM & POVAL CO., LTD. (polymerization degree: 1480, saponification degree: 22.7%)) was dissolved into ethanol/1-methoxy-2-propanol (7/3 by mass) so that the concentration thereof would be 3 mg/g. A composition for forming a coating film 2 having an ability to suppress adhesion of cells was thus prepared. The composition obtained was transparent and uniform.

<Preparation Example 3>

46.6 g of pure water was added to 0.0466 g of the polymer obtained in Synthesis Example 1, and the mixture was thoroughly stirred to give a diluent composition.

<Production Example 1>

(Production of substrate having an ability to suppress adhesion of cells)

The coating film-forming composition 1 of Preparation Example 1 was added to each of the wells of a 24-well cell culture plate (#351147 manufactured by Corning, 1 mL volume, made of polystyrene) at 25 μL/well, and was allowed to stand at room temperature for 3 hours and was dried in an oven at 70°C for 24 hours. A substrate 1 having an ability to suppress adhesion of cells was thus produced.

<Production Example 2>

(Production of substrate having an ability to suppress adhesion of cells)

The coating film-forming composition 1 of Preparation Example 1 was added to each of the wells of a 24-well cell culture plate (#351147 manufactured by Corning, 1 mL volume, made of polystyrene) at 100 μL/well, and was allowed to stand at room temperature for 3 hours and was dried in an oven at 70°C for 24 hours. A substrate 2 having an ability to suppress adhesion of cells was thus produced.

<Production Example 3>

(Production of substrate having an ability to suppress adhesion of cells)

The coating film-forming composition 1 of Preparation Example 1 was added to each of the wells of a Φ40 mm Petri dish (#1-8549-01 manufactured by AS ONE Corporation, 1 mL volume, made of polystyrene) at 125 μL/well, and was allowed to stand at room temperature for 3 hours and was dried in an oven at 70°C for 24 hours. A substrate 3 having an ability to suppress adhesion of cells was thus produced.

<Production Example 4>

(Production of substrate having an ability to suppress adhesion of cells by inkjet printer)

With use of an inkjet device (inkjet device for R&D manufactured by Seiko Epson Corporation) and an inkjet head (Precision Core Head S800-A1 manufactured by Seiko Epson Corporation), an appropriate amount of the coating film-forming composition 2 prepared in Preparation Example 2 was applied to draw a perfect circle having a diameter of 18 mm on a polystyrene substrate having a size of 79 mm × 121 mm. The coating was dried in an oven at 70°C for 24 hours. A substrate 4 having an ability to suppress adhesion of cells was thus produced.

<Production Example 5>

(Production of substrate having an ability to suppress adhesion of cells by inkjet printer)

With use of an inkjet device (inkjet device for R&D manufactured by Seiko Epson Corporation) and an inkjet head (Precision Core Head S800-A1 manufactured by Seiko Epson Corporation), an appropriate amount of the coating film-forming composition 2 prepared in Preparation Example 2 was applied to draw a perfect circle having a diameter of 37 mm on a polystyrene substrate having a size of 79 mm × 121 mm. The coating was dried in an oven at 70°C for 24 hours. A substrate 5 having an ability to suppress adhesion of cells was thus produced.

<Example 1>

99.5 g of pure water and 0.5 mL of 0.5 mg/mL vitronectin VTN-N (Gibco) were added to 0.0025 g of the polymer obtained in Synthesis Example 1, and the mixture was thoroughly stirred to give a base film-forming agent. With use of an inkjet device (model number: LaboJet-600 manufactured by MICROJET) and an inkjet head (model number: 500-S-C), an appropriate amount of the base film-forming agent was applied to the culture surface of a culture dish having an ability to suppress adhesion of cells (diameter: 35 mm) (Sumitomo Bakelite Co., Ltd., MS9035X) to form a large number of spots. The spots were dried in a constant temperature dryer at 70°C for 1 day. A cell aggregate production substrate was thus produced.

<Example 2>

99 g of pure water and 1 mL of 0.5 mg/mL vitronectin VTN-N (Gibco) were added to 0.0025 g of the polymer obtained in Synthesis Example 1, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Example 3>

99 g of pure water and 1.0 mL of 0.5 mg/mL vitronectin VTN-N (Gibco) were added to 0.005 g of the polymer obtained in Synthesis Example 2, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Example 4>

99 g of pure water and 1.0 mL of 0.5 mg/mL vitronectin VTN-N (Gibco) were added to 0.005 g of the polymer obtained in Synthesis Example 1, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Example 5>

94 g of pure water and 6.0 mL of 0.5 mg/mL vitronectin VTN-N (Gibco) were added to 0.015 g of the polymer obtained in Synthesis Example 1, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of a culture plate having an ability to suppress adhesion of cells (PrimeSurface (registered trademark) Plate 24F, model number: MS-90240, Sumitomo Bakelite Co., Ltd.) to form a large number of spots having a diameter of 250 μm. The spots were dried in a constant temperature dryer at 70°C for 1 day. A cell aggregate production substrate was thus produced. Sterilization was performed by applying 25 kGy of gamma rays.

<Example 6>

98 g of pure water and 2.0 mL of 0.5 mg/mL vitronectin VTN-N (Gibco) were added to 0.005 g of the polymer obtained in Synthesis Example 1, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of a culture plate having

an ability to suppress adhesion of cells (PrimeSurface (registered trademark) Plate 24F, model number: MS-90240, Sumitomo Bakelite Co., Ltd.) to form a large number of spots having a diameter of 250 μm. The spots were dried in a constant temperature dryer at 70°C for 1 day. A cell aggregate production substrate was thus produced. Sterilization was performed by applying 25 kGy of gamma rays.

<Example 7>

31.3 g of pure water and 2.0 mL of 0.5 mg/mL vitronectin VTN-N (Gibco) were added to 0.005 g of the polymer obtained in Synthesis Example 1, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture plate having an ability to suppress adhesion of cells (PrimeSurface (registered trademark) Plate 24F, model number: MS-90240, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Example 8>

3.69 g of sterile water and 0.28 g of Recombinant Human Vitronectin (manufactured by Peprotech) diluted to 0.5 mg/mL with sterile water were added to 0.70 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of the substrate 4 having an ability to suppress adhesion of cells produced in Production Example 4 to form a large number of spots having a diameter of 400 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 9>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 4 having an ability to suppress adhesion of cells produced in Production Example 4 to form a large number of spots having a diameter of 400 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 10>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 4 having an ability to suppress adhesion of cells produced in Production Example 4. After drying was performed at room temperature for 5 minutes, the agent was applied again to form an overcoat. A large number of spots having a diameter of 400 μm were thus formed. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 11>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 5 having an ability to suppress adhesion of cells produced in Production Example 5 to form a large number of spots having a diameter of 400 μm. The spots were dried in a constant temperature dryer at 70°C for 1 day. The substrate was affixed to a bottomless 6-well plate (manufactured by CSTEC Corporation). A cell aggregate production substrate was thus produced. Sterilization was performed by applying 25 kGy of gamma rays.

<Example 12>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 5 having an ability to suppress adhesion of cells produced in Production Example 5. After drying was performed at room temperature for 5 minutes, the agent was applied again to form an overcoat. A large number of spots having a diameter of 400 μm were thus formed. The spots were dried in a constant temperature dryer at 70°C for 1 day. The substrate was affixed to a bottomless 6-well plate (manufactured by CSTEC Corporation). A cell aggregate production substrate was thus produced. Sterilization was performed by applying 25 kGy of gamma rays.

<Example 13>

17.9 g of sterile water and 0.60 g of Recombinant Human Vitronectin (manufactured by Peprotech) diluted to 0.5 mg/mL with sterile water were added to 1.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of the substrate 4 having an ability to suppress adhesion of cells produced in Production Example 4 to form a large number of spots having a diameter of 400 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 14>

4.48 g of sterile water and 0.08 g of iMatrix-511 (manufactured by MATRIXOME) were added to 0.80 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of the substrate 2 having an ability to suppress adhesion of cells produced in Production Example 2 to form a large number of spots having a diameter of 400 μm. The spots were dried in a constant temperature dryer at 70°C for 1 day. A cell aggregate production substrate was thus produced. Sterilization was performed by applying 25 kGy of gamma rays.

<Example 15>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 14 was applied to the culture surface of the substrate 4 having an ability to suppress adhesion of cells produced in Production Example 4 to form a large number of spots having a diameter of 400 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 16>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 14 was applied to the culture surface of the substrate 4 having an ability to suppress adhesion of cells produced in Production Example 4. After drying was performed at room temperature for 5 minutes, the agent was applied again to form an overcoat. A large number of spots having a diameter of 400 μm were thus formed. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 17>

5.59 g of sterile water and 0.10 g of iMatrix-221 (manufactured by MATRIXOME) were added to 1.00 g of the diluent composition obtained in Preparation Example 3, and the mixture was thoroughly stirred to give a base film-forming

agent. With use of an inkjet device (model number: LaboJet-600 manufactured by MICROJET) and an inkjet head (model number: IJHBS-1000), an appropriate amount of the base film-forming agent was applied to the culture surface of the substrate 2 having an ability to suppress adhesion of cells produced in Production Example 2 to form a large number of spots having a diameter of 400 μm. The spots were dried in a constant temperature dryer at 70°C for 1 day. A cell aggregate production substrate was thus produced. Sterilization was performed by applying 25 kGy of gamma rays.

<Example 18>

7.54 g of sterile water and 0.30 g of 0.5 mg/mL human plasma-derived fibronectin solution (manufactured by FUJIFILM Wako Pure Chemical Corporation) purified with a spin column (Zeba Spin Desalting Columns and Plates, 7K MWCO, manufactured by Thermo Fisher Scientific K.K.) were added to 0.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of a culture plate having an ability to suppress adhesion of cells (PrimeSurface (registered trademark) Plate 24F, model number: MS-90240, Sumitomo Bakelite Co., Ltd.) to form a large number of spots having a diameter of 400 μm. The spots were dried in a constant temperature dryer at 70°C for 1 day. A cell aggregate production substrate was thus produced. Sterilization was performed by applying 25 kGy of gamma rays.

<Example 19>

33.35 g of sterile water and 1.28 g of 0.5 mg/mL vitronectin VTN-N (Gibco) purified with a spin column (Zeba Spin Desalting Columns and Plates, 7K MWCO, manufactured by Thermo Fisher Scientific K.K.) were added to 2.81 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of a culture plate having an ability to suppress adhesion of cells (PrimeSurface (registered trademark) Plate 24F, model number: MS-90240, Sumitomo Bakelite Co., Ltd.) to form a large number of spots having a diameter of 400 μm. The spots were dried in a constant temperature dryer at 70°C for 1 day. A cell aggregate production substrate was thus produced. Sterilization was performed by applying 25 kGy of gamma rays.

<Example 20>

13.92 g of sterile water and 0.26 g of peptide RGDS (model number: 4171-v, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example21>

13.92 g of sterile water and 0.25 g of peptide GRGDS (model number: 4189, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example 22>

13.92 g of sterile water and 0.25 g of peptide GRGDNP (model number: PCI-3909-PI, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model

number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example 23>

13.92 g of sterile water and 0.25 g of peptide YIGSR-NH2 (model number: 4194-v, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example 24>

13.92 g of sterile water and 0.25 g of peptide c [RGDfK (C)] (model number: RGD-3794-PI, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example 25>

14.12 g of sterile water and 0.05 g of peptide c [RGDfK (C)] (model number: RGD-3794-PI, PEPTIDE INSTITUTE, INC.) diluted to 0.5 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of the substrate 3 having an ability to suppress adhesion of cells produced in Production Example 3 to form a large number of spots. The spots were dried in a constant temperature dryer at 70°C for 1 day. A cell aggregate production substrate was thus produced. Sterilization was performed by applying 25 kGy of gamma rays.

<Example 26>

30.33 g of sterile water and 0.50 g of PASAP1 (manufactured by Nanomed3D) diluted to 1.0 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of the substrate 1 having an ability to suppress adhesion of cells produced in Production Example 1 to form a large number of spots having a diameter of 400 μm. The spots were dried in a constant temperature dryer at 70°C for 1 day. A cell aggregate production substrate was thus produced. Sterilization was performed by applying 25 kGy of gamma rays.

<Example 27>

30.33 g of sterile water and 0.51 g of PASAP2 (manufactured by Nanomed3D) diluted to 1.0 mg/mL with sterile water were added to 2.50 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: IJHBS-1000), an appropriate amount of the base film-forming agent was applied to the culture surface of the substrate 1 having an ability to suppress adhesion of cells produced in Production Example 1 to form a large number of spots having a diameter of 400 μm. The spots were dried in a constant temperature

dryer at 70°C for 1 day. A cell aggregate production substrate was thus produced. Sterilization was performed by applying 25 kGy of gamma rays.

<Example 28>

3.45 g of sterile water, 0.23 g of ethanol, and 0.28 g of Recombinant Human Vitronectin (manufactured by Peprotech) diluted to 0.5 mg/mL with sterile water were added to 0.70 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture dish having an ability to suppress adhesion of cells (diameter: 35 mm) (MS9035X, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Example 29>

3.22 g of sterile water, 0.47 g of ethanol, and 0.28 g of Recombinant Human Vitronectin (manufactured by Peprotech) diluted to 0.5 mg/mL with sterile water were added to 0.70 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture dish having an ability to suppress adhesion of cells (diameter: 35 mm) (MS9035X, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Example 30>

3.70 g of sterile water, 0.28 g of Recombinant Human Vitronectin (manufactured by Peprotech) diluted to 0.5 mg/mL with sterile water, and 0.0056 g of a tannic acid (manufactured by FUJIFILM Wako Pure Chemical Corporation) solution diluted to 10 mg/mL with sterile water were added to 0.70 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture dish having an ability to suppress adhesion of cells (diameter: 35 mm) (MS9035X, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Example31>

4.21 g of sterile water, 0.32 g of Recombinant Human Vitronectin (manufactured by Peprotech) diluted to 0.5 mg/mL with sterile water, and 0.0063 g of a gallic acid hydrate (manufactured by Tokyo Chemical Industry Co., Ltd.) solution diluted to 10 mg/mL with sterile water were added to 0.80 g of the diluent composition obtained in Preparation Example 3, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture dish having an ability to suppress adhesion of cells (diameter: 35 mm) (MS9035X, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Example 32>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 4 having an ability to suppress adhesion of cells produced in Production Example 4 so as to form spots having a diameter of 170 μm and a center-to-center interval between the spots of 250 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 33>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 4 having an ability to suppress adhesion of cells produced in Production Example 4 so as to form spots having a diameter of 400 μm and a center-to-center interval between the spots of 500 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Example 34>

With use of an inkjet device (inkjet device for R&D manufactured by Seiko Epson Corporation) and an inkjet head (Precision Core Head S800-A1 manufactured by Seiko Epson Corporation), an appropriate amount of the base film-forming agent prepared in Example 8 was applied to the culture surface of the substrate 4 having an ability to suppress adhesion of cells produced in Production Example 4 so as to form spots having a diameter of 900 μm and a center-to-center interval between the spots of 1000 μm. The spots were dried in a constant temperature dryer at 70°C for 1 day. The substrate was affixed to a bottomless 24-well plate (manufactured by CSTEC Corporation). A cell aggregate production substrate was thus produced. Sterilization was performed by applying 25 kGy of gamma rays.

<Comparative Example 1>

100.0 g of pure water was added to 0.0025 g of the polymer obtained in Synthesis Example 1, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Comparative Example 2>

100.0 g of pure water was added to 0.005 g of the polymer obtained in Synthesis Example 2, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Comparative Example 3>

33.3 g of pure water was added to 0.005 g of the polymer obtained in Synthesis Example 1, and the mixture was thoroughly stirred to give a base film-forming agent. With use of an inkjet device (model number: LaboJet-600 manufactured by MICROJET) and an inkjet head (model number: 200-S-C), an appropriate amount of the base film-forming agent was applied to the culture surface of a culture dish having an ability to suppress adhesion of cells (diameter: 35 mm) (MS9035X, Sumitomo Bakelite Co., Ltd.) to form a large number of spots. The spots were dried in a constant temperature dryer at 70°C for 1 day. A cell aggregate production substrate was thus produced. Sterilization was performed by applying 25 kGy of gamma rays.

<Comparative Example 4>

4.8 g of pure water and 0.2 mL of 0.5 mg/mL vitronectin VTN-N (Gibco) were added, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Comparative Example 5>

2.1 g of pure water and 1.2 mL of 0.5 mg/mL vitronectin VTN-N (Gibco) were added, and the mixture was thoroughly stirred to give a base film-forming agent. The base film-forming agent was applied using the inkjet device to form a large number of spots, and the spots were dried in the same manner as in Example 1. A cell aggregate production substrate was thus produced.

<Test Example 1: Cell adhesion confirmation test of Examples 1 to 3 and Comparative Examples 1 and 2 with mouse fibroblasts in FBS-free media>

(Preparation of cells)

Mouse fetal fibroblasts (C3H10T1/T2 cells, manufactured by DS Pharma Biomedical Co., Ltd.) were used as cells. The medium for cell culture was one prepared by adding 10% FBS (manufactured by Sigma-Aldrich) and 1% glutamine/penicillin/streptomycin (manufactured by Gibco) to BME medium (manufactured by Gibco) as a basal medium. The cells were statically cultured in a 10 cm diameter Petri dish (10 mL medium) in a 37°C/$CO_2$ incubator for at least 2 days while maintaining the carbon dioxide concentration at 5%. Subsequently, the cells were washed with 3 mL of PBS solution (manufactured by FUJIFILM Wako Pure Chemical Corporation), and 3 mL of trypsin-EDTA solution (manufactured by PromoCell) was added. The mixture was allowed to stand at room temperature for 3 minutes, and the cells were detached. 7 mL of BME medium free of FBS (bovine serum) and glutamine/penicillin/streptomycin was added, and the cells were harvested. The suspension obtained was centrifuged (model number: LC-230 manufactured by TOMY SEIKO CO., LTD., 200 $\times$ g/3 minutes, room temperature). The supernatant was removed. The above medium was added. A cell suspension was thus prepared.

(Cell adhesion confirmation test)

2.0 mL of the cell suspension was added to the cell aggregate production substrates produced in Examples 1 to 3 and Comparative Examples 1 and 2. The seeding was performed so that the cell density would be 1.5 $\times$ $10^5$ cells/$cm^2$ in Example 1, Example 2, and Comparative Example 1, and the cell density would be 3.0 $\times$ $10^5$ cells/$cm^2$ in Example 3 and Comparative Example 2. Subsequently, the cells were allowed to stand in a 37°C/$CO_2$ incubator for 2 hours while maintaining the carbon dioxide concentration at 5%. After the incubation, the free cells and the medium were removed, and the wells were washed with PBS to leave only the adherent cells on the wells. After the washing, 2.0 mL of new medium was added, and the appearance of the adherent cells was observed and photographed with stereomicroscope SZX16 (manufactured by Olympus Corporation). As illustrated in Fig. 1, the observation confirmed that the cells had adhered selectively to the base film portions on the substrates produced in Examples 1 to 3 and Comparative Examples 1 and 2. In Examples 1 to 3, the cells had adhered uniformly without any gaps. In contrast, the cell adhesion in Comparative Examples 1 and 2 contained gaps and was nonuniform.

The above results have shown that uniform cell adhesion can be achieved on the base film in a serum (FBS)-free medium when the base film-forming agent contains an additive that promotes the adhesion and the stretching of cells.

<Test Example 2: Cell adhesion-cell aggregate formation confirmation test of Examples 4 and 6 with mouse fibroblasts in FBS-free media>

(Preparation of cells)

Cells were prepared in the same manner as in Test Example 1.

(Cell adhesion confirmation test)

2.0 mL of the cell suspension was added to the cell aggregate production substrates produced in Examples 4 and 6 so that the cell density would be 3.0 $\times$ $10^5$ cells/$cm^2$. Subsequently, the cells were allowed to stand in a 37°C/$CO_2$ incubator for 2 hours while maintaining the carbon dioxide concentration at 5%. After the incubation, the free cells and the medium were removed, and the wells were washed with PBS to leave only the adherent cells on the wells. After the washing, 2.0 mL of the medium was newly added, and the appearance of the adherent cells was observed and photographed with stereomicroscope SZX16 (manufactured by Olympus Corporation). Furthermore, the presence or absence of cell aggregate formation was checked and photographed the next day. As illustrated in Fig. 2, the observation confirmed that the cells had adhered selectively to the base film portions on the substrates. The cells had adhered uniformly without any gaps. The observation after 2 days confirmed that the adherent cells had separated from the Petri dish and had aggregated to form cell aggregates (spheroids). The above results have shown that the base film containing an additive that promotes the adhesion and the stretching of cells allows cells to adhere uniformly thereto and to separate therefrom later to form cell aggregates.

<Test Example 3: Cell adhesion confirmation test of Example 5 and Comparative Example 3 with human adipose tissue-derived mesenchymal stem cells in serum-free media>

(Preparation of cells)

Human adipose tissue-derived mesenchymal stem cells (ADSC manufactured by CellSource Co., Ltd.) were used as cells. Low-serum medium Mesenchymal Stem Cell Growth Medium 2 (manufactured by Takara Bio Inc., serum concentration 2%) was used for cell culture. The cells were statically cultured in a 10 cm diameter Petri dish (10 mL medium) for at least 2 days in a 37°C/$CO_2$ incubator while maintaining the carbon dioxide concentration at 5%. Subsequently, the cells were washed with 3 mL of PBS solution (manufactured by FUJIFILM Wako Pure Chemical Corporation), and 3 mL of trypsin-EDTA solution (manufactured by PromoCell) was added. The mixture was allowed to stand at room temperature for 3 minutes, and the cells were detached. 7 mL of serum-free Mesenchymal Stem Cell Growth Medium DXF was added, and the cells were harvested. The suspension obtained was centrifuged (model number: LC-230 manufactured by TOMY SEIKO CO., LTD., 200 $\times$ g/3 minutes, room temperature). The supernatant was removed. The above medium was added. A cell suspension was thus prepared.

(Cell adhesion confirmation test)

2.0 mL of the cell suspension was added to the cell aggregate production substrates produced in Example 5 and Comparative Example 3 so that the cell density would be $3.0 \times 10^5$ cells/cm$^2$. Subsequently, the cells were allowed to stand in a 37°C/$CO_2$ incubator for 2 hours while maintaining the carbon dioxide concentration at 5%. After the incubation, the free cells and the medium were removed, and the wells were washed with PBS to leave only the adherent cells on the wells. After the washing, 2.0 mL of the medium was newly added, and the appearance of the adherent cells was observed and photographed with stereomicroscope SZX16 (manufactured by Olympus Corporation). As illustrated in Fig. 3, the observation confirmed that the cells had adhered selectively to the base film portions on the substrate produced in Example 5. Furthermore, the cells had adhered uniformly without any gaps. In contrast, the cell adhesion in Comparative Example 3 contained gaps and was nonuniform.

The above results have shown that uniform ADSC cell adhesion can be achieved on the base film in a serum-free medium when the base film-forming agent contains an additive that promotes the adhesion and the stretching of cells.

<Test Example 4: Cell adhesion confirmation test of Example 7 with human adipose tissue-derived mesenchymal stem cells in low-serum medium>

(Preparation of cells)

Cells were prepared in the same manner as in Test Example 3, except that the culture medium after cell detachment was changed to low-serum Mesenchymal Stem Cell Growth Medium 2.

(Cell adhesion confirmation test)

The cell aggregate production substrate produced in Example 7 was subjected to a cell adhesion confirmation test in the same manner as in Test Example 3. As illustrated in Fig. 4, the observation confirmed that the cells had adhered selectively to the base film portions on the substrate. Furthermore, the cells had adhered uniformly without any gaps.

The above results have shown that uniform ADSC cell adhesion can be achieved on the base film in a low-serum medium when the base film-forming agent contains an additive that promotes the adhesion and the stretching of cells.

<Test Example 5: Cell adhesion-cell aggregate formation confirmation test of Examples 8 to 10, 13, 14, 17 to 19, 26, and 27 with human adipose tissue-derived mesenchymal stem cells in serum-free media>

(Preparation of cells)

Cells were prepared in the same manner as in Test Example 3.

(Cell adhesion-cell aggregate formation confirmation test)

0.5 to 2.0 mL of the cell suspension was added to the cell aggregate production substrates produced in Examples

8 to 10, 13, 14, 17 to 19, 26, and 27 so that the cell density would be $2.0 \times 10^5$ cells/$cm^2$. Subsequently, the cells were allowed to stand in a 37°C/$CO_2$ incubator for 2 hours while maintaining the carbon dioxide concentration at 5%. After the incubation, the appearance of the adherent cells was observed and photographed with stereomicroscope SZX16 (manufactured by Olympus Corporation). As illustrated in Fig. 5, the observation confirmed that the cells had adhered selectively to the base film portions on the substrates. The cells had adhered uniformly without any gaps. The observation after 2 days confirmed that the adherent cells had separated from the Petri dish and had aggregated to form cell aggregates (spheroids). The above results have shown that the base film containing an additive that promotes the adhesion and the stretching of cells allows cells to adhere thereto uniformly and to separate therefrom later to form cell aggregates.

<Test Example 6: Cell adhesion-cell aggregate formation confirmation test of Examples 20 to 25 and 28 to 31 with human adipose tissue-derived mesenchymal stem cells in serum-free media>

(Preparation of cells)

Cells were prepared in the same manner as in Test Example 3.

(Cell adhesion-cell aggregate formation confirmation test)

2.0 mL of the cell suspension was added to the cell aggregate production substrates produced in Examples 20 to 25 and 28 to 31 so that the cell density would be $2.0 \times 10^5$ cells/$cm^2$. Subsequently, the cells were allowed to stand in a 37°C/$CO_2$ incubator for 2 hours while maintaining the carbon dioxide concentration at 5%. After the incubation, the free cells and the medium were removed, and the wells were washed with PBS to leave only the adherent cells on the wells. After the washing, 2.0 mL of the medium was newly added, and the appearance of the adherent cells was observed and photographed with stereomicroscope SZX16 (manufactured by Olympus Corporation). As illustrated in Fig. 6, the observation confirmed that the cells had adhered selectively to the base film portions on the substrates. The cells had adhered uniformly without any gaps. The observation after 1 day confirmed that the adherent cells had separated from the Petri dish and had aggregated to form cell aggregates (spheroids). The above results have shown that the base film containing an additive that promotes the adhesion and the stretching of cells allows cells to adhere thereto uniformly and to separate therefrom later to form cell aggregates.

<Test Example 7: Cell adhesion-cell aggregate formation confirmation test of Examples 32 to 34 with human adipose tissue-derived mesenchymal stem cells in serum-free media>

(Preparation of cells)

Cells were prepared in the same manner as in Test Example 3.

(Cell adhesion confirmation test)

1.0 mL of the cell suspension was added to the cell aggregate production substrates produced in Examples 32 to 34 so that the cell density would be $0.75 \times 10^5$ to $3.0 \times 10^5$ cells/$cm^2$. Subsequently, the cells were allowed to stand in a 37°C/$CO_2$ incubator for 6 hours while maintaining the carbon dioxide concentration at 5%. After the incubation, the appearance of the adherent cells was observed and photographed with stereomicroscope SZX16 (manufactured by Olympus Corporation). Furthermore, the presence or absence of cell aggregate formation was checked and photographed 2 days later. As illustrated in Fig. 7, the observation confirmed that the cells had adhered selectively to the base film portions on the substrates. The cells had adhered uniformly without any gaps. The diameter of the adherent cells was the same as the diameter of the coating. The observation after 2 days confirmed that the adherent cells had separated from the Petri dish and had aggregated to form cell aggregates (spheroids). Furthermore, the observation showed that the diameter of the cell aggregates changed depending on the adhesion diameter. The above results have shown that the base film containing an additive that promotes the adhesion and the stretching of cells allows cells to adhere thereto uniformly and to separate therefrom later to form cell aggregates. The results have also shown that the diameter of adherent cells and the diameter of cell aggregates can be controlled by controlling the diameter of the coating.

<Test Example 8: Cell adhesion confirmation test of Examples 9 and 10 with human induced pluripotent stem cells>

(Preparation of cells)

Human induced pluripotent stem cell (hiPSC) 1383D2 line (obtained from Center for iPS Cell Research and Application, Kyoto University) was cultured with mTeSR1 medium (manufactured by VERITAS Corporation) for at least 3 days in a 6-well plate coated with 0.5 μg/cm$^2$ Vitronectin (VTN-N) Recombinant Human Protein, Truncated (manufactured by Thermo Fisher Scientific K.K.) in a 37°C/5% $CO_2$ incubator. Subsequently, the cells were washed with 2 mL/well of PBS solution (manufactured by FUJIFILM Wako Pure Chemical Corporation), and 0.5 mL of TrypLE (trademark) Select Enzyme (1X), no phenol red (manufactured by Thermo Fisher Scientific K.K.) was added. After the incubation at room temperature for 10 minutes, the cells were detached as single cells. The cells were recovered using mTeSR1 medium, and the suspension was centrifuged (model number: EIX-136, manufactured by TOMY SEIKO CO., LTD., 200 × g/3 minutes, room temperature). The supernatant was removed. The cells were suspended in mTeSR1 medium containing 10 μM Y-27632 (manufactured by FUJIFILM Wako Pure Chemical Corporation). The suspension was passed through a 40 μm cell strainer (manufactured by Corning) for preparation of single cell suspension.

(Cell adhesion confirmation test)

0.5 mL of the cell suspension was seeded at a density of 1.5 × 10$^5$ cells/cm$^2$ on the cell aggregate production substrates produced in Examples 9 and 10.

Subsequently, the cells were statically cultured in a 37°C/5% $CO_2$ incubator for 3 hours or 24 hours. After the incubation, the non-adherent cells and the medium were removed, and the wells were washed with mTeSR1 medium to leave only the adherent cells on the wells. After the washing, 0.5 mL of new medium was added, and the adherent cells was observed and photographed with inverted microscope IX73 (manufactured by Olympus Corporation). It was confirmed that hiPSC had adhered to the plate as illustrated in Fig. 8.

The above results have shown that uniform hiPSC cell adhesion on the base film can be achieved when the base film-forming agent contains an additive that promotes the adhesion and the stretching of cells.

<Test Example 9: Spheroid formation confirmation test of Examples 9 and 10 with human induced pluripotent stem cells>

0.5 mL of the cell suspension prepared in Test Example 8 was seeded at a density of 1.5 × 10$^5$ cells/cm$^2$ on the cell aggregate production substrates produced in Examples 9 and 10. Subsequently, the cells were statically cultured in a 37°C/5% $CO_2$ incubator. On the day after the seeding, the non-adherent cells and the medium were removed, and 0.5 mL of mTeSR1 medium without Y-27632 was added to replace new medium without Y-27632. The cells were further statically cultured in the 37°C/5% $CO_2$ incubator. Two days after the seeding, the cells was observed and photographed with inverted microscope IX73 (manufactured by Olympus Corporation). As illustrated in Fig. 9, it was confirmed that the adherent cells had detached from the plate and had formed cell aggregates (spheroids). The uniform sizes of the spheroids were confirmed in all the wells.

<Test Example 10: Viability and undifferentiation confirmation test of Examples 11 and 12 after cell adhesion and spheroid formation using human induced pluripotent stem cells>

Human induced pluripotent stem cell (hiPSC) 1383D2 line was cultured with mTeSR1 and prepared single cells suspension in the same manner as in Test Example 8.

2 mL of the cell suspension was seeded at a density of 1.5 × 10$^5$ cells/cm$^2$ on the cell aggregate production substrates produced in Examples 11 and 12. Subsequently, the cells were statically cultured in a 37°C/5% $CO_2$ incubator. The day after the seeding, the non-adherent cells and the medium were removed, and 2 mL of mTeSR1 medium without Y-27632 was added to replace new medium without Y-27632. The cells were further statically cultured in the 37°C/5% $CO_2$ incubator.

Two days after the seeding, the cell aggregates (spheroids) were formed in the same manner as in Test Example 9. The spheroids were recovered in a 15 mL centrifuge tube and were centrifuged at 200 × g for 3 minutes. The supernatant was removed, and the spheroids was suspended in 0.25 mL of TrypLE (trademark) Select Enzyme (1X), no phenol red (manufactured by Thermo Fisher Scientific K.K.). The spheroids were dispersed as single cells by pipetting while incubating in a water bath at 37°C for 5 to 10 minutes. The reaction of TrypLE was stopped by adding 750 μL of DMEM containing 10% FBS, and the cells were counted with Nucleocounter NC-200 (manufactured by Chemometec). As described in Table 1, the cell viability of iPSC spheroids was remained high.

[Table 1]

| Example | Viability |
|---|---|
| Example 11 | 96.1% |
| Example 12 | 95.3% |

Subsequently, the cells that had been dispersed as single cells were washed with SM buffer (2% FBS/PBS), and PE-labeled mouse anti-human SSEA-4 antibody and AlexaFluor 647-labeled mouse anti-human TRA-1-60 antibody (manufactured by BD Biosciences) were added. The cells were incubated at room temperature for 30 minutes, washed twice with SM buffer, and analyzed with FACS LSRFortessa X-20 (manufactured by BD Biosciences). As described in Table 2, iPSC formed spheroids were co-expressed SSEA-4 and TRA-1-60 in a high proportion, which indicated that the iPSC formed spheroids by this method maintained undifferentiated.

[Table 2]

| | Example | Proportion of SSEA-4 (+)/ TRA-1-60 (+) cells |
|---|---|---|
| Day 0 | - | 99.2% |
| Day 2 | Example 11 | 99.3% |
| Day 2 | Example 12 | 99.3% |

<Test Example 11: Cell adhesion confirmation test of Examples 15 and 16 with human induced pluripotent stem cells>

0.5 mL of the cell suspension was seeded at a density of $1.5 \times 10^5$ cells/cm$^2$ on the cell aggregate production substrates produced in Examples 15 and 16. Subsequently, the cells were statically cultured in a 37°C/5% $CO_2$ incubator for 6 hours, and the adherent cells was observed and photographed with inverted microscope IX73 (manufactured by Olympus Corporation). As illustrated in Fig. 10, the hiPSC adhered to these plates were confirmed. Subsequently, the cells were statically cultured in the 37°C/5% $CO_2$ incubator until 26 hours after the seeding. After the incubation, the non-adherent cells and the medium were removed, and the wells were washed with mTeSR1 medium to leave only the adherent cells on the wells. After the washing, 0.5 mL of new medium was added, and the adherent cells was observed and photographed with inverted microscope IX73 (manufactured by Olympus Corporation). As illustrated in Fig. 10, the hiPSC adhered to these plates were confirmed.

The above results have shown that uniform hiPSC cell adhesion on the base film can be achieved when the base film-forming agent contains an additive that promotes the adhesion and the stretching of cells.

<Test Example 12: Spheroid formation confirmation test of Examples 15 and 16 with human induced pluripotent stem cells>

0.5 mL of the cell suspension was seeded at a density of $1.5 \times 10^5$ cells/cm$^2$ on the cell aggregate production substrates produced in Examples 15 and 16. Subsequently, the cells were statically cultured in a 37°C/5% $CO_2$ incubator. On the day after the seeding, the non-adherent cells and the medium were removed, and 0.5 mL of mTeSR1 medium without Y-27632 was added to replace new medium without Y-27632. The cells were further statically cultured in the 37°C/5% $CO_2$ incubator.

Two days after the seeding, the cells was observed and photographed with inverted microscope IX73 (manufactured by Olympus Corporation). As illustrated in Fig. 11, it was confirmed that the adherent cells had detached from the plates and had formed cell aggregates (spheroids). The uniform sizes of the spheroids were confirmed in all the wells.

<Test Example 13: Cell adhesion-cell aggregate formation confirmation test of Comparative Example 4 (polymer-free, additive only) with mouse fibroblasts in FBS-free medium>

(Preparation of cells)

Cells were prepared in the same manner as in Test Example 1.

(Cell adhesion confirmation test)

The cell aggregate production substrate produced in Comparative Example 4 was subjected to a cell adhesion-cell aggregate formation confirmation test in the same manner as in Test Example 2. As illustrated in Fig. 12, the observation did not confirm cell adhesion to the base film portions on the substrate. This result shows that cell adhesion cannot be achieved when the base film is free of polymers and contains only an additive that promotes the adhesion and the stretching of cells. Thus, it has been found that the cell adhesion effect is produced only when the polymer and the additive are combined.

<Test Example 14: Cell adhesion-cell aggregate formation confirmation test of Comparative Example 5 (polymer-free, additive only) with mouse fibroblasts in FBS-containing medium>

(Preparation of cells)

Cells were prepared in the same manner as in Test Example 1, except that the culture medium after cell detachment was changed to BME medium containing 10% FBS (bovine serum) and 1% glutamine/penicillin/streptomycin.

(Cell adhesion confirmation test)

The cell aggregate production substrate produced in Comparative Example 5 was subjected to a cell adhesion-cell aggregate formation confirmation test in the same manner as in Test Example 2. As illustrated in Fig. 13, the observation confirmed that the cells had adhered selectively to the base film portions on the substrate. The cells had adhered uniformly without any gaps. The observation after 2 days confirmed that the adherent cells remained fixed on the Petri dish. The above results have shown that the base film that is free of polymers and contains only an additive that promotes the adhesion and the stretching of cells allows cells to adhere thereto uniformly in a serum medium, but the cells are not allowed to separate later and fail to form cell aggregates.

INDUSTRIAL APPLICABILITY

The cell culture base film-forming agent of the present invention allows cells to adhere thereto uniformly and later to form cell aggregates regardless of whether an animal-derived serum is present or absent in the culture conditions. Thus, the use of the cell culture base film-forming agent realizes mass production of homogeneous and high-quality cell aggregates for use in the field of regenerative medicine.

## Claims

1. A base film-forming agent for cell culture comprising a polymer, a cell adhesive substance, and a solvent, wherein the polymer contains a repeating unit derived from a monomer of by formula (I) below:

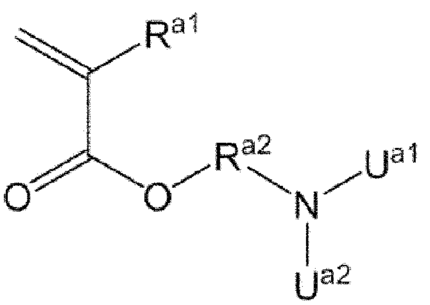

(I)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group.

2. The base film-forming agent for cell culture according to claim 1, wherein the polymer further contains a repeating unit derived from a monomer of formula (II):

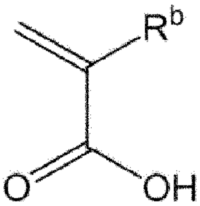

(II)

wherein
$R^b$ denotes a hydrogen atom or a C1-C5 linear or branched alkyl group.

3. The base film-forming agent for cell culture according to claim 1 or 2, wherein the weight ratio of the polymer to the cell adhesive substance is 100:0.1 to 100: 100.

4. The base film-forming agent for cell culture according to any one of claims 1 to 3, wherein the cell adhesive substance comprises a glycoprotein.

5. A substrate for producing cell aggregate comprising spots including a base film for cell culture formed, on a substrate having an ability to suppress adhesion of cells, by the base film-forming agent for cell culture according to any one of claims 1 to 4.

6. A method for producing cell aggregate comprising a step of forming, on a substrate having an ability to suppress adhesion of cells, a base film for cell culture comprising a polymer and a cell adhesive substance, wherein the polymer contains a repeating unit derived from a monomer of formula (I) below:

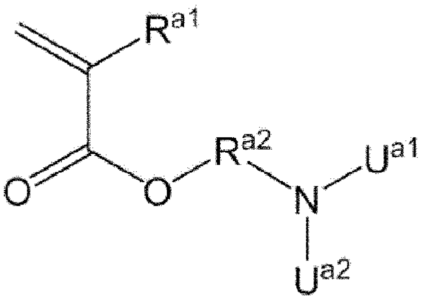

(I)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group; and a step of seeding a cell.

7. A base film-forming agent for cell culture comprising a polymer, a cell adhesive substance, and a solvent, wherein the polymer contains a repeating unit of formula (Ia) below and a crosslink structure:

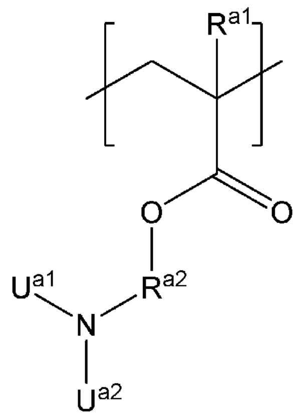

(Ia)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group.

**8.** The base film-forming agent for cell culture according to claim 7, wherein the crosslink structure comprises a structure derived from a polyfunctional acrylate compound, a polyfunctional acrylamide compound, a polyfunctional polyester, or an isoprene compound.

**9.** The base film-forming agent for cell culture according to claim 7, wherein the crosslink structure comprises a structure of formula (IIIa), formula (IVa), and/or formula (Va) below:

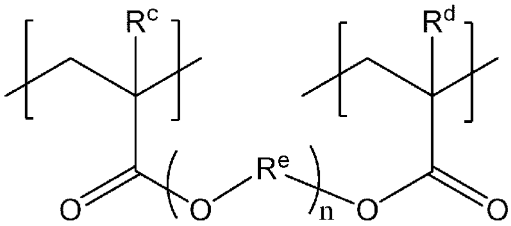
(IIIa)

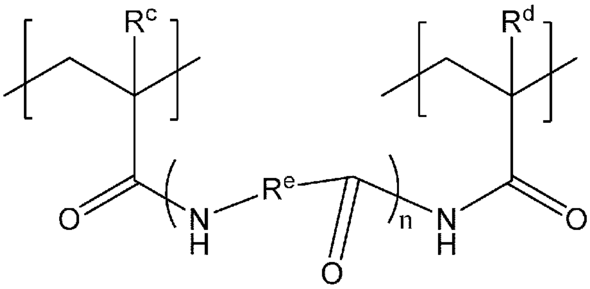
(IVa)

and/or

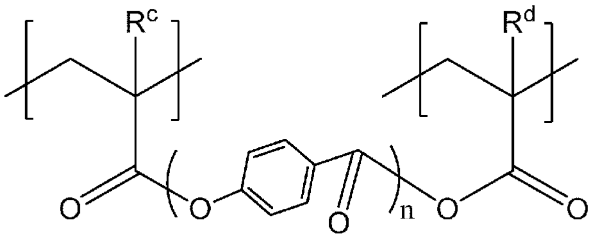
(Va)

wherein $R^c$ and $R^d$ each independently denote a hydrogen atom or a C1-C5 linear or branched alkyl group, $R^e$ denotes a C1-C5 linear or branched alkylene group, and n denotes a number of 1 to 50.

**10.** A method for producing cell aggregate comprising a step of forming, on a substrate having an ability to suppress adhesion of cells, a base film for cell culture comprising a polymer and a cell adhesive substance, wherein the polymer contains a repeating unit of formula (Ia) below and a crosslink structure:

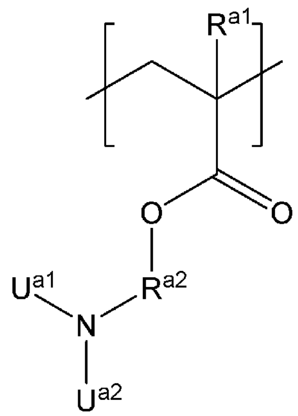

(Ia)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group; and a step of seeding a cell.

**11.** A method for producing cell aggregate comprising a step of seeding a cell to a substrate for producing cell aggregate comprising a base film for cell culture comprising a polymer and a cell adhesive substance on a substrate having an ability to suppress adhesion of cells, wherein the polymer contains a repeating unit of formula (Ia) below and a crosslink structure:

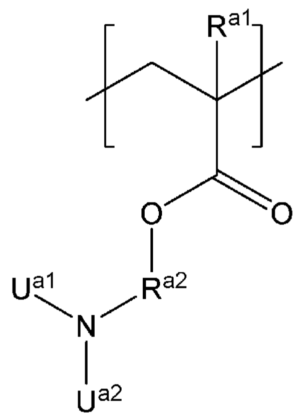

(Ia)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group.

**12.** A substrate for producing cell aggregate comprising spots including a base film for cell culture comprising a polymer and a cell adhesive substance, wherein the polymer contains a repeating unit of formula (Ia) below and a crosslink structure:

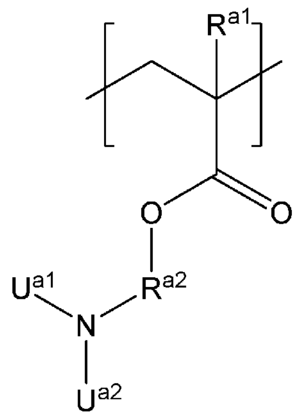

(Ia)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group.

**13.** A base film-forming agent for cell culture comprising a polymer, a cell adhesive substance, and a solvent, wherein the polymer contains a repeating unit of formula (Ia) below, a repeating unit of formula (IIa) below, and a crosslink structure:

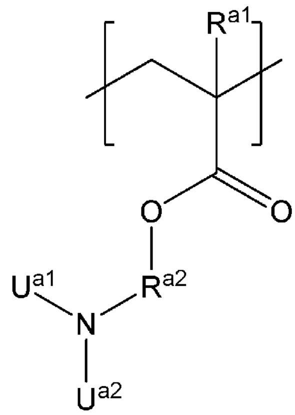

(Ia)

wherein
$U^{a1}$ and $U^{a2}$ each independently denote a hydrogen atom or a C1-C5 linear or branched alkyl group, $R^{a1}$ denotes a hydrogen atom or a C1-C5 linear or branched alkyl group, and $R^{a2}$ denotes a C1-C5 linear or branched alkylene group,

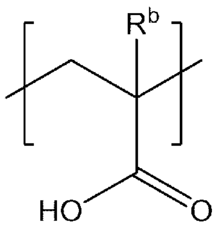

(IIa)

wherein
$R^b$ denotes a hydrogen atom or a C1-C5 linear or branched alkyl group.

Fig. 1

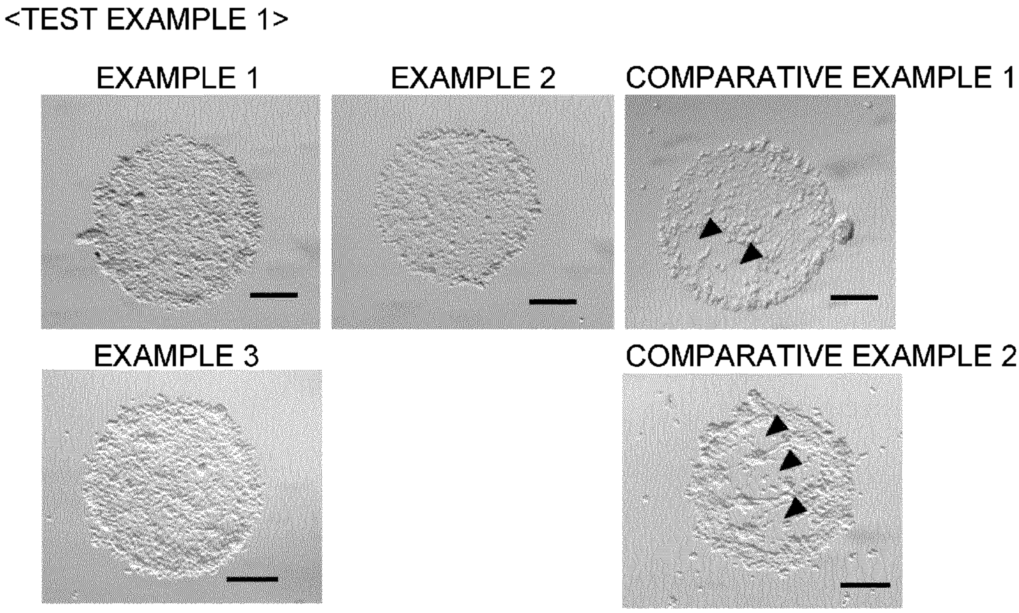


Fig. 2

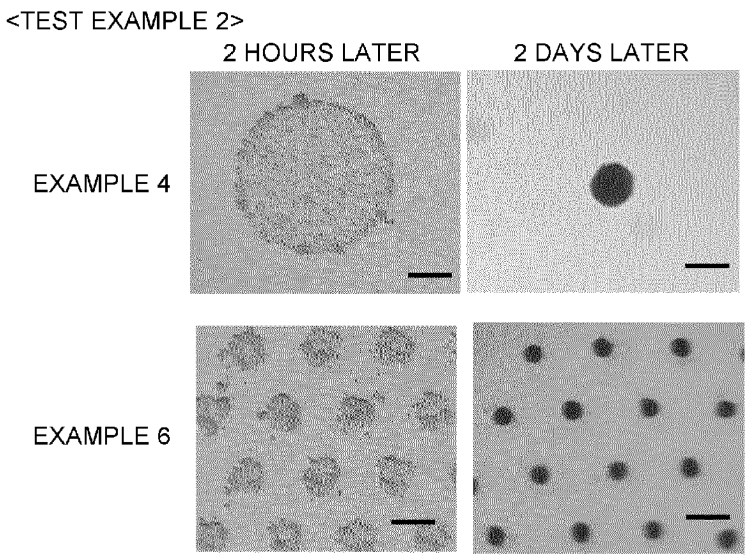


Fig. 3

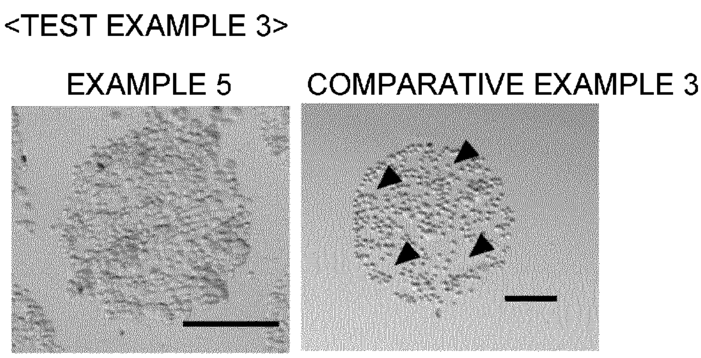

Fig. 4

<TEST EXAMPLE 4>

EXAMPLE 7

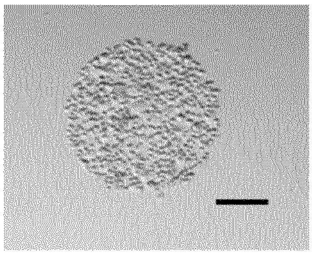

Fig. 5-1

<TEST EXAMPLE 5>

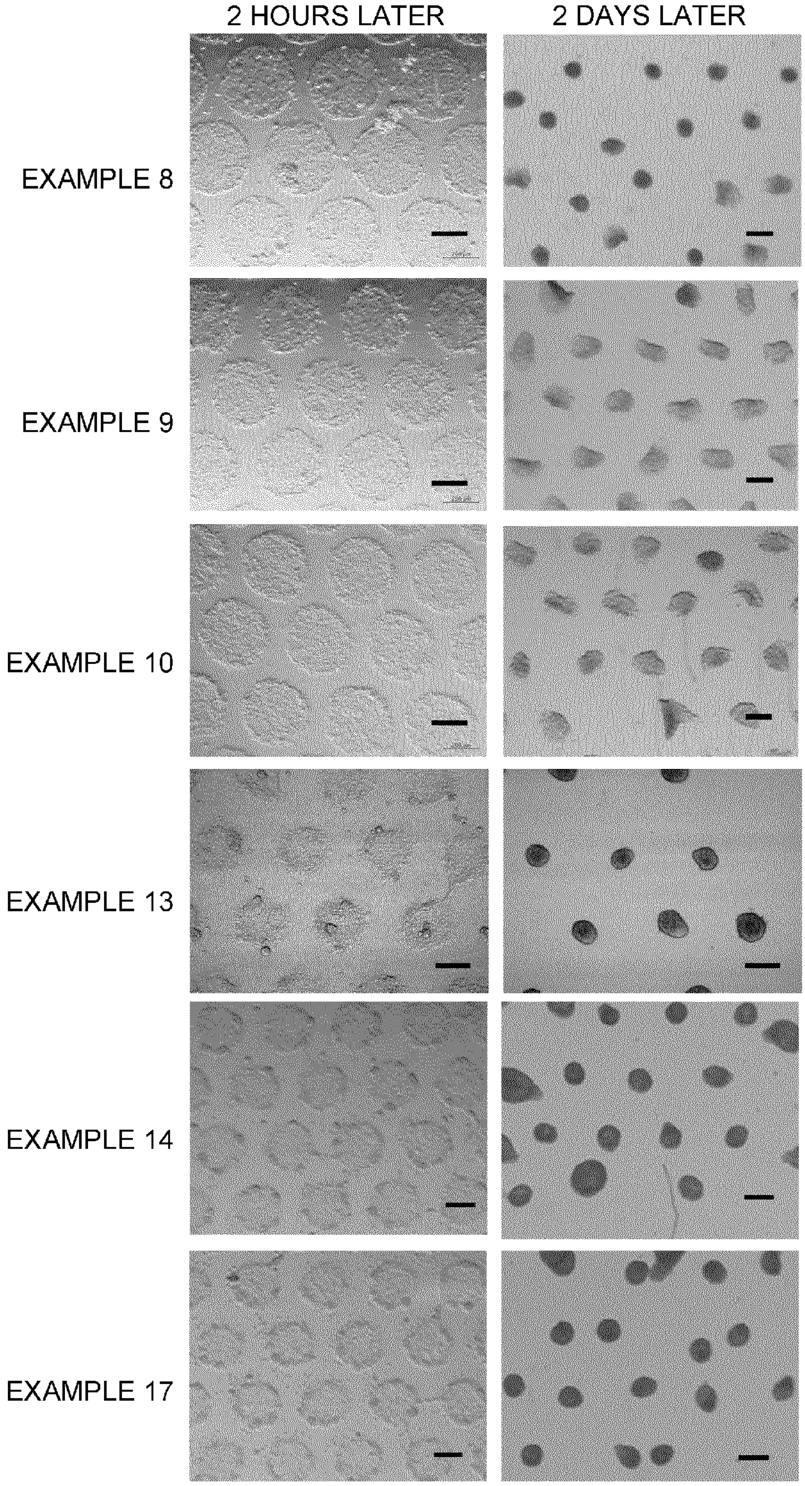

Fig. 5-2

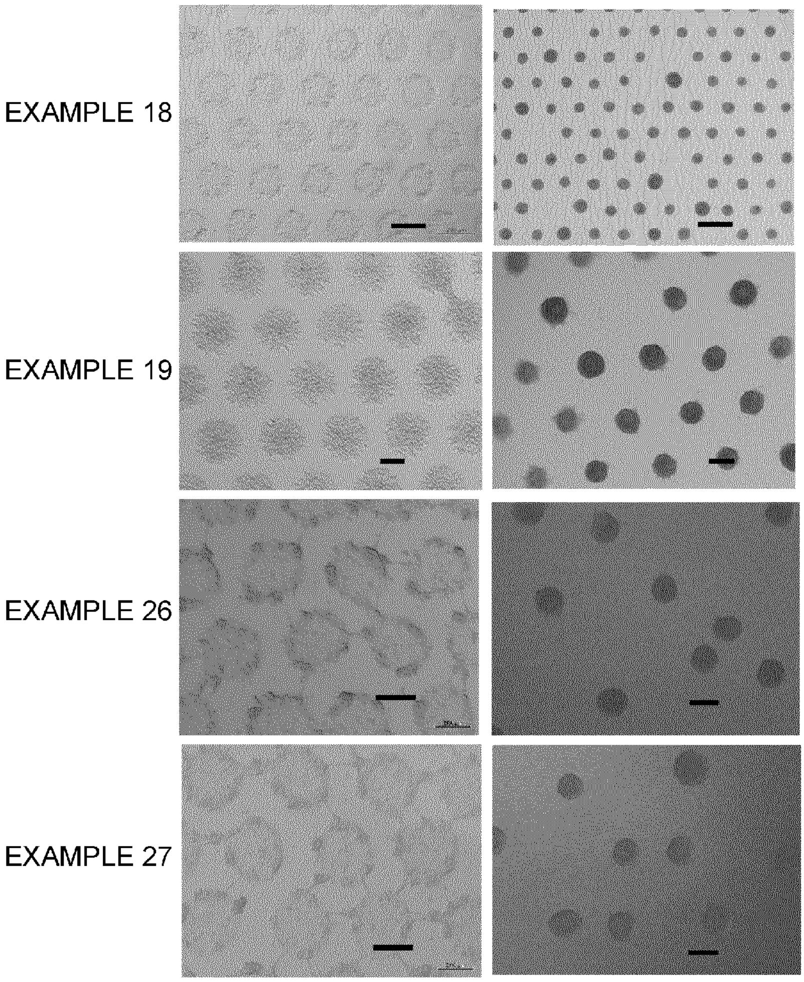

Fig. 6-1

<TEST EXAMPLE 6>

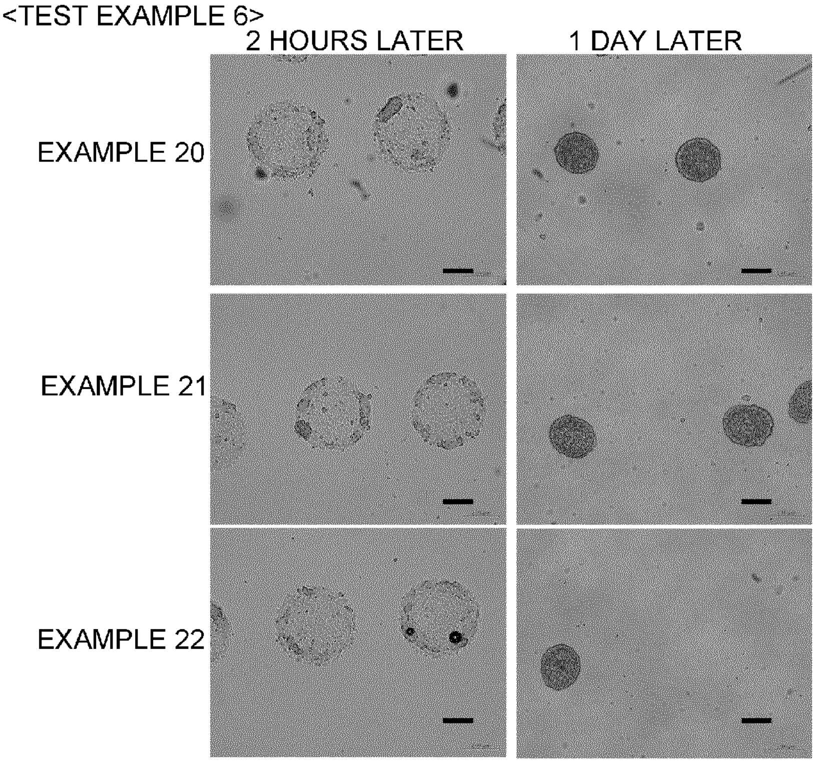

Fig. 6-2

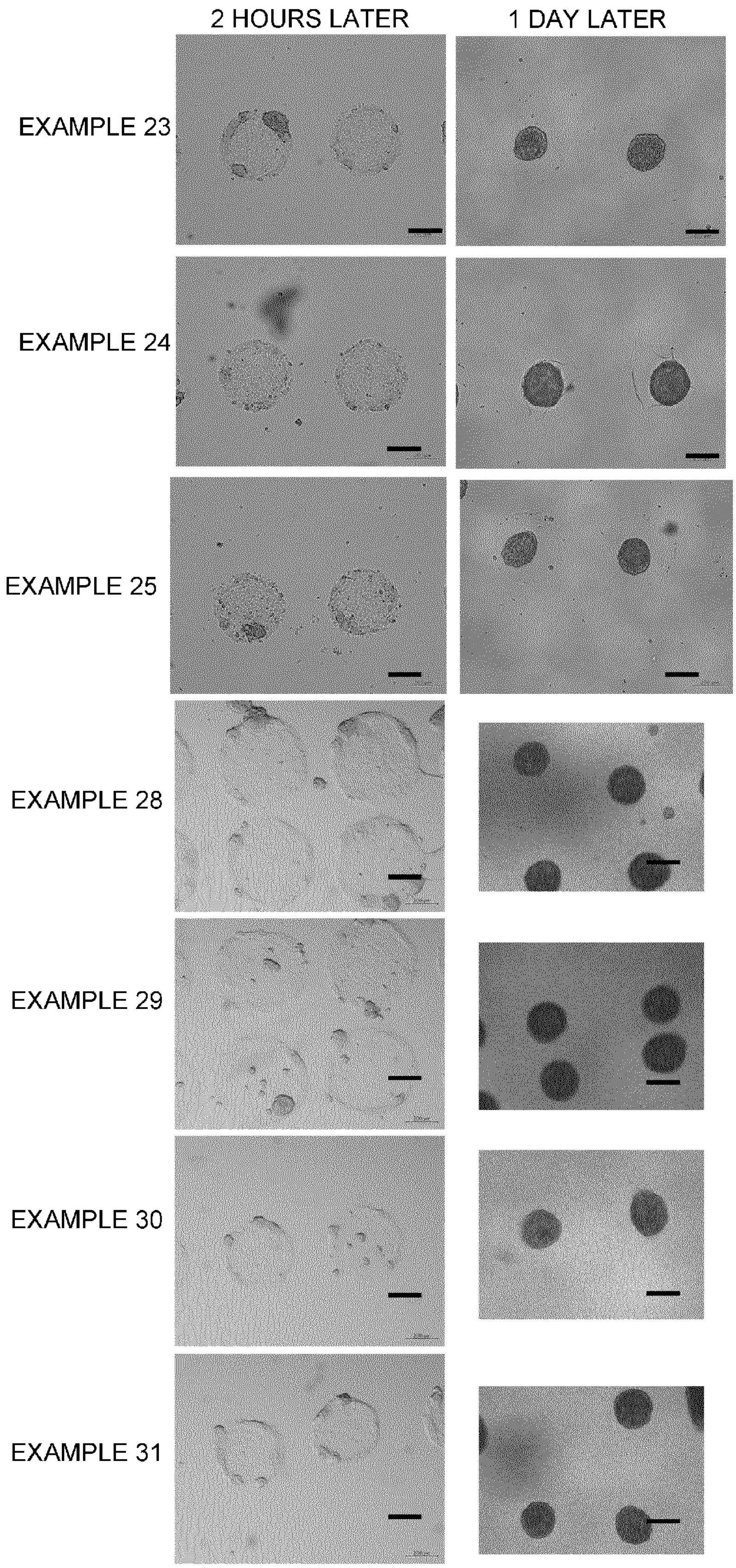
2 HOURS LATER
1 DAY LATER
EXAMPLE 23
EXAMPLE 24
EXAMPLE 25
EXAMPLE 28
EXAMPLE 29
EXAMPLE 30
EXAMPLE 31

Fig. 7

<TEST EXAMPLE 7>

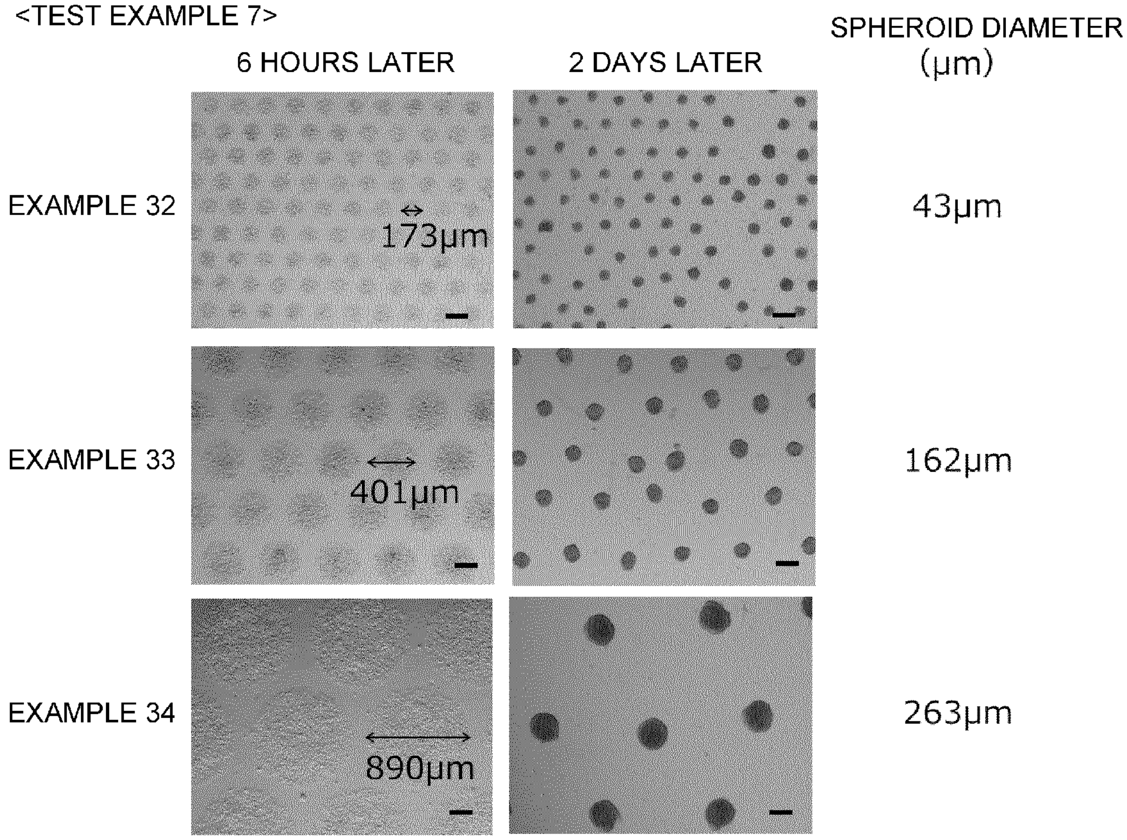


Fig. 8

<TEST EXAMPLE 8>

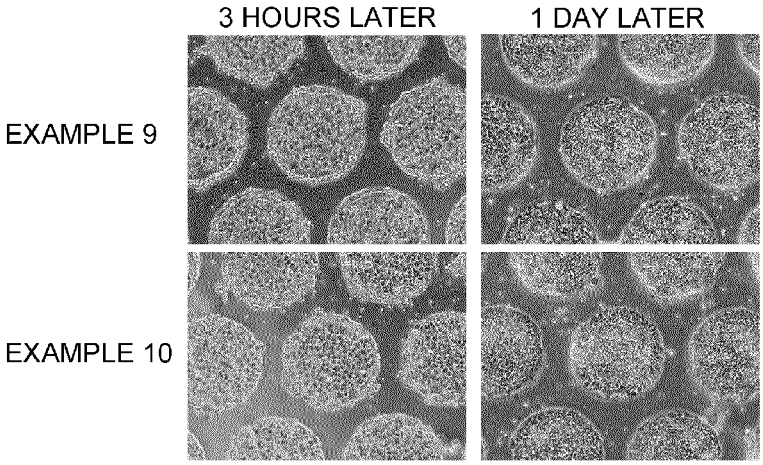

Fig. 9

<TEST EXAMPLE 9>

EXAMPLE 9 | EXAMPLE 10

2 DAYS LATER

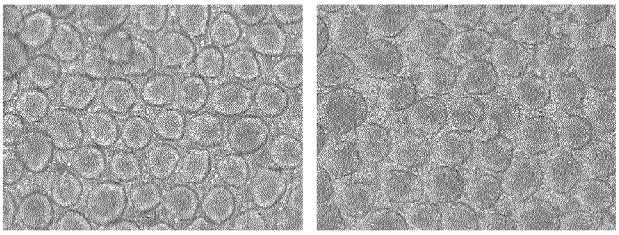

Fig. 10

<TEST EXAMPLE 11>

6 HOURS LATER | 1 DAY LATER

EXAMPLE 15

EXAMPLE 16

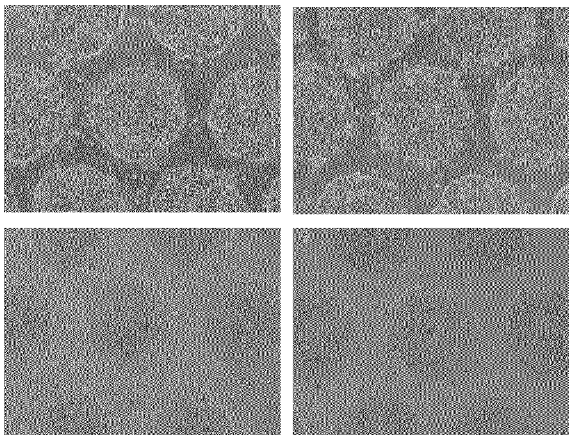

Fig. 11

<TEST EXAMPLE 12>

EXAMPLE 15 | EXAMPLE 16

2 DAYS LATER

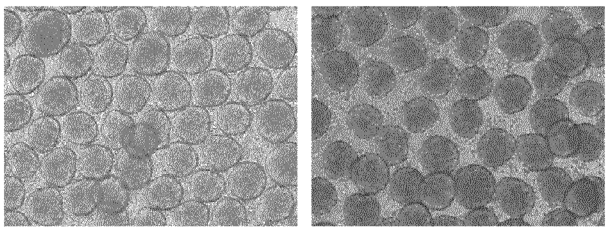

Fig. 12

<TEST EXAMPLE 13>

2 HOURS LATER | 2 DAYS LATER

COMPARATIVE EXAMPLE 4

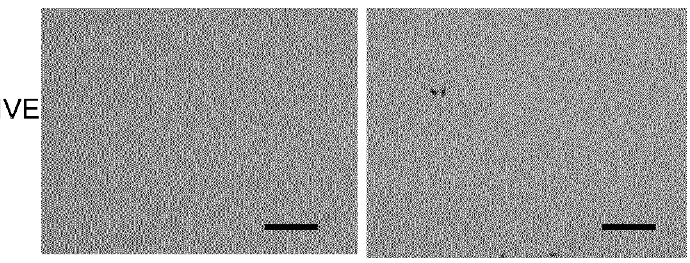

Fig. 13

<TEST EXAMPLE 14>

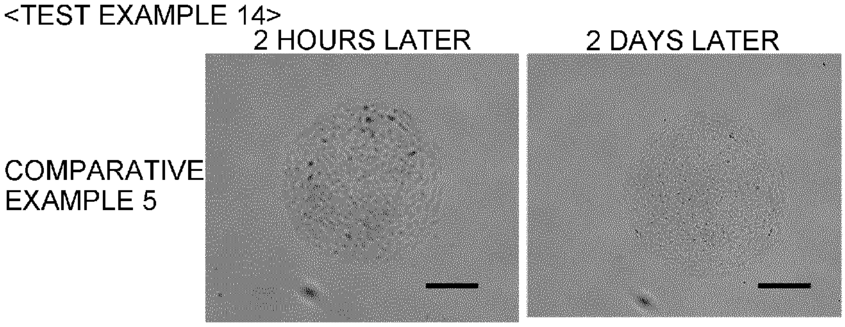

## INTERNATIONAL SEARCH REPORT

International application No.
**PCT/JP2022/026688**

**A. CLASSIFICATION OF SUBJECT MATTER**

***C12N 5/071***(2010.01)i; ***C08F 20/34***(2006.01)i; ***C12M 1/00***(2006.01)i
FI: C12N5/071; C08F20/34; C12M1/00 C

According to International Patent Classification (IPC) or to both national classification and IPC

**B. FIELDS SEARCHED**

Minimum documentation searched (classification system followed by classification symbols)

C12N5/071; C08F20/34; C12M1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 5-292957 A (KURABO IND LTD) 09 November 1993 (1993-11-09)<br>claims, paragraphs [0003], [0016], [0021], [0026] | 1-4, 7, 13<br>5-6, 8-12 |
| A | WO 2020/040247 A1 (NISSAN CHEMICAL CORPORATION) 27 February 2020 (2020-02-27)<br>claims, paragraphs [0040]-[0044] | 1-13 |
| A | JP 2014-162865 A (NATIONAL CEREBRAL & CARDIOVASCULAR CENTER) 08 September 2014 (2014-09-08)<br>claims | 1-13 |
| A | JP 2021-91872 A (TOYO INK SC HOLDINGS CO LTD) 17 June 2021 (2021-06-17)<br>claims, paragraphs [0018], [0032]-[0034] | 1-13 |
| A | WO 2014/196650 A1 (NISSAN CHEMICAL INDUSTRIES, LTD.) 11 December 2014 (2014-12-11)<br>claims, paragraphs [0041], [0079]-[0084] | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 August 2022** | **13 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/026688**

| Patent document cited in search report | Publication date (day/month/year) | Patent family member(s) | Publication date (day/month/year) |
|---|---|---|---|
| JP 5-292957 A | 09 November 1993 | US 5643561 A<br>claims, column 1, lines 30-41, column 4, lines 23-30, column 7, lines 29-35, column 9, lines 2-9<br>EP 567886 A2 | |
| WO 2020/040247 A1 | 27 February 2020 | US 2021/0238329 A1<br>claims, paragraphs [0063]-[0065]<br>EP 3842519 A1 | |
| JP 2014-162865 A | 08 September 2014 | (Family: none) | |
| JP 2021-91872 A | 17 June 2021 | (Family: none) | |
| WO 2014/196650 A1 | 11 December 2014 | US 2016/0122576 A1<br>claims, paragraphs [0057], [0101]-[0104]<br>EP 3006529 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020040247 A
- JP 2017143755 A
- JP 2020028120 A
- JP 2008061609 A
- WO 2014196650 A
- WO 2016093293 A
- JP 2010169604 A
- JP 2008533489 A
- JP 2014048278 A